# EUROPEAN PATENT SPECIFICATION

(11) **EP 1 812 599 B1**
(45) Date of publication and mention of the grant of the patent: **23.09.2009**
(21) Application number: 05815286.9
(22) Date of filing: 12.10.2005
(51) Int. Cl.: C12Q 1/68

(54) **METHODS AND COMPOSITIONS FOR ANALYSING RIBONUCLEIC ACIDS**
METHODEN UND ZUSAMMENSETZUNGEN ZUR ANALYSE VON RIBONUKLEINSÄUREN
PROCEDES ET COMPOSITIONS PERMETTANT D'ANALYSER DES ACIDES RIBONUCLEIQUES

(30) Priority: 12.10.2004 US 963415
(43) Date of publication of application: 01.08.2007
(73) Proprietor: ASURAGEN, INC., Austin, TX 78744 (US)
(72) Inventor: WINKLER, Matthew, M., Austin, TX 78746 (US); GOLDRICK, Marianna, Austin, TX 78704 (US); HARRIS, Nathan, Austin, TX 78745 (US); YE, Fei, Austin, TX 78749 (US)
(74) Representative: Sonn & Partner Patentanwälte
(86) International application number: PCT/US2005/036799
(87) International publication number: WO 2006/042303

(56) References cited:
- US-A- 5 660 988
- US-A1- 2003 204 322
- US-A1- 2004 072 164
- LEE YOONTAE ET AL: "The nuclear RNase III Drosha initiates microRNA processing." NATURE (LONDON), vol. 425, no. 6956, 25 September 2003 (2003-09-25), pages 415-419, XP002384567 ISSN: 0028-0836
- XIE ZHIXIN ET AL: "Negative feedback regulation of Dicer-Like1 in Arabidopsis by microRNA-guided mRNA degradation." CURRENT BIOLOGY : CB. 29 APR 2003, vol. 13, no. 9, 29 April 2003 (2003-04-29), pages 784-789, XP002384568 ISSN: 0960-9822
- LIU CHANG-GONG ET AL: "An oligonucleotide microchip for genome-wide microRNA profiling in human and mouse tissues" PROCEEDINGS OF THE NATIONAL ACADEMY OF SCIENCES OF USA, NATIONAL ACADEMY OF SCIENCE, WASHINGTON, DC, US, vol. 101, no. 26, 29 June 2004 (2004-06-29), pages 9740-9744, XP002364908 ISSN: 0027-8424
- ALLAWI HATIM T ET AL: "Quantitation of microRNAs using a modified Invader assay" RNA (COLD SPRING HARBOR), vol. 10, no. 7, July 2004 (2004-07), pages 1153-1161, XP002384569 ISSN: 1355-8382
- KIM ET AL: "Genomics of microRNA" TRENDS IN GENETICS, ELSEVIER SCIENCE PUBLISHERS B.V. AMSTERDAM, NL, vol. 22, no. 3, March 2006 (2006-03), pages 165-173, XP005308293 ISSN: 0168-9525

### SEQUENCE LISTING

<110> WINKLER, MATTHEW M.
   GOLDRICK, MARIANNA
   HARRIS, NATHAN
   YE, FEI
<120> METHODS AND COMPOSITIONS FOR ANALYZING NUCLEIC ACIDS
<130> AMBI:101WO
<140> UNKNOWN
   <141> 2005-10-12
<150> 10/963,415
   <151> 2004-10-12
<160> 14
<170> PatentIn Ver. 2.1
<210> 1
   <211> 22
   <212> DNA
   <213> Artificial Sequence
<220>
   <223> Description of Artificial Sequence: Synthetic Primer
<400> 1
   uagcagcacg uaaauauugg cg 22
<210> 2
   <211> 22
   <212> DNA
   <213> Artificial Sequence
<220>
   <223> Description of Artificial Sequence: Synthetic Primer
<400> 2
   cgccaauauu uacgugcugc ua 22
<210> 3
   <211> 22
   <212> DNA
   <213> Artificial Sequence
<220>
   <223> Description of Artificial Sequence: Synthetic Primer
<400> 3
   cgccaatatt tacgugcugc ua 22
<210> 4
   <211> 71
   <212> DNA
   <213> Artificial Sequence
<220>
   <223> Description of Artificial Sequence: Synthetic Primer
<400> 4
   cttttcccgt ccgtcatcgc tcaagtggca ttcaccgcgu gccttaactc cctatagtga 60
   gtcgtattac g                                                       71
<210> 5
   <211> 15
   <212> DNA
   <213> Artificial Sequence
<220>
   <223> Description of Artificial Sequence: Synthetic Primer
<400> 5
   ctcaagtggc attca 15
<210> 6
   <211> 19
   <212> DNA
   <213> Artificial Sequence
<220>
   <223> Description of Artificial Sequence: Synthetic Primer
<400> 6
   cttttcccgt ccgtcatcg 19
<210> 7
   <211> 20
   <212> DNA
   <213> Artificial Sequence
<220>
   <223> Description of Artificial Sequence: Synthetic Primer
<400> 7
   ctatagggag ttaaggcacg 20
<210> 8
   <211> 71
   <212> DNA
   <213> Artificial Sequence
<220>
   <223> Description of Artificial Sequence: Synthetic Primer
<400> 8
   cttttcccgt ccgtcatcgc tcaagtggca uucaccgcgt gccttaactc cctatagtga 60
   gtcgtattac g 71
<210> 9
   <211> 19
   <212> DNA
   <213> Artificial Sequence
<220>
   <223> Description of Artificial Sequence: Synthetic Primer
<400> 9
   gctcaagtgg cattcaccg 19
<210> 10
   <211> 18
   <212> DNA
   <213> Artificial Sequence
<220>
   <223> Description of Artificial Sequence: Synthetic Primer
<400> 10
   cttttcccgt ccgtcatc 18
<210> 11
   <211> 20
   <212> DNA
   <213> Artificial Sequence
<220>
   <223> Description of Artificial Sequence: Synthetic Primer
<400> 11
   ctatagggag ttaaggcacg 20
<210> 12
   <211> 22
   <212> DNA
   <213> Artificial Sequence
<220>
   <223> Description of Artificial Sequence: Synthetic Primer
<400> 12
   ttaaggcacg cggtgaatgc ca 22
<210> 13
   <211> 22
   <212> DNA
   <213> Artificial Sequence
<220>
   <223> Description of Artificial Sequence: Synthetic Primer
<400> 13
   ttaaggcacg cggtcaatgc ca 22
<210> 14
   <211> 20
   <212> DNA
   <213> Artificial Sequence
<220>
   <223> Description of Artificial Sequence: Synthetic Primer
<400> 14
   taatacgact cactataggg 20

## Description

### BACKGROUND OF THE INVENTION

The United States government may own rights in the present invention pursuant to grant number R44 CA88699 from the National Institutes of Health (NIH).

### 1. Field of the Invention

The present invention is in the field of molecular biology. More specifically, the present invention concerns methods and compositions for probe amplification to detect and/or quantify target nucleic acids in a sample and allows for some particular benefits in regard to the detection and/or quantification of nucleic acids that are present in relatively small amounts or that are relatively small in size.

### 2. Description of the Related Art

Many types of studies carried out on biological samples require the quantitative detection of ribonucleic acid species, including messenger RNA (mRNA), micro RNA (miRNA) and small interfering RNA (siRNA) molecules. miRNAs are a recently-discovered class of short single-stranded RNA molecules that are - 22 bases in length, and which are partially complementary to sequences in the 3' untranslated region of mRNAs; miRNAs serve to inhibit expression of their target mRNAs by post-transcriptional mechanisms which are not completely understood (Pasquinelli and Ruvkun, 2002). Typical methods for quantitative detection of mRNA include Northern blot analysis, RNase protection assay (RPA), and various embodiments of the polymerase chain reaction (PCR™) for example, reverse - transcription polymerase chain reaction (RT-PCR), and quantitative (also known as real-time) RT-PCR (qRT-PCR). These methods are however inadequate or inconvenient for detection of very short RNAs, including miRNA and siRNA targets, because such RNA species, being only ~ 21 - 23 bases in length, are too short to be specifically primed (as is required for RT-PCR-based detection) and are poorly resolved on the gel matrices typically used for Northern blot and RPA detection. Conventional methods for RNA detection and quantitation have additional drawbacks as described below.

Different techniques and reagents exist for identifying, detecting, isolating, or quantifying a specific nucleic acid, such as a specific RNA molecule. However, they each have distinct advantages, as well as disadvantages, and consequently, new methods are continually developed to address advancements in the field of molecular biology and the desires for efficiency, accuracy, safety, and cost-savings.

Traditionally, gene expression has been studied using techniques based on hybridization of labeled oligonucleotide probes to specific target mRNAs in samples consisting of total RNA extracted from cells or tissue. In the most commonly used method, Northern Blot analysis, the total RNA is fractionated prior to hybridization. Fractionation is carried out by electrophoresis of sample RNA on an agarose or polyacrylamide gel under denaturing conditions. The size-separated RNA is then transferred to a solid support such as a nitrocellulose or nylon membrane (a process referred to as "blotting"), and the RNA on the solid support is then contacted for several hours, (typically overnight) with a solution containing the labeled probe (a process referred to as "hybridizing the probe to the blot"). Prior to contacting the size-fractionated RNA with the probe, the membrane is generally treated for several hours ("prehybridized") by contacting it with the solution without the probe, to block non-specific binding of the probe. After the probe hybridization step, the membrane is washed extensively to remove unhybridized probe, and then treated in a way to detect the remaining labeled probe, which is specifically hybridized to the target RNA. See Ausubel, 1994 and Sambrook, 1989 and 2001 (2nd and 3rd editions), all of which are specifically incorporated by reference.

Common methods for labeling the probe are by enzymatic incorporation of radioisotopically modified nucleotides (*e.g*., [³²P]-NTPs or [³²P]-dNTPs) or of nucleotides modified to contain other detectable moieties such as biotin or digoxigenin. Probes may also be labeled post-synthetically for example using crosslinking agents such as psoralen to introduce detectable moieties into the probe. Detection of radiolabeled probes is typically accomplished by exposing the membrane to X-ray film for hours or days, or by use of a phosphorimaging instrument. Detection of non-isotopically labeled probes involves a multistep process of contacting and incubating the membrane with a series of solutions for generating luminescent or chemiluminescent signals, washing the membrane to reduce background, and exposing the blot to film.

Variations on the Northern Blot technique include dot-blots, reverse dot-blots, slot-blots, etc. and in general involve the same series of steps with the exception that the sample RNA is not fractionated prior to hybridization. Northern Blot and related methods are labor-intensive and time-consuming, as each step (gel electrophoresis, blotting, prehybridization, hybridization, and detection) typically requires one to several hours or longer. Northern blots also suffer from a requirement for highly intact target RNA (since a positive result depends detecting a signal at the appropriate position on the membrane for a target mRNA of the expected size). This requirement precludes the use of samples, that may be compromised due to lag time in harvesting the RNA or due to prior treatments such as aldehyde fixation, which are known to damage RNA. Another major drawback to Northern blotting and other methods that do not include a target amplification step is that they are relatively insensitive. Northern blotting may lack the sensitivity required for detection of low-abundance targets and for detecting targets in very small mass amounts of sample RNA such as those obtained from microdissected tissue. For example, Kim *et al.* speculate that lack of sensitivity may explain the failure to detect 23 of 40 newly-discovered candidate miRNAs in rat brain using Northern blotting (Kim *et al.,* 2004).

Another traditional non-amplification-based method for detecting mRNA expression is the RNase Protection Assay (RPA) as e.g. described by Lee et al., Nature 425; 2003: 415-419. This method is based on hybridizing a labeled RNA probe to a biological sample that may contain the target mRNA, then treating the reaction with single-strand-specific RNase to digest unhybridized probe. Probe that is hybridized to target RNA is double-stranded and therefore "protected" from RNase digestion. The protected probe is recovered and detected, the amount of protected probe being proportional to the amount of target RNA in the sample. Labeled probes are typically made by *in vitro* transcription using a phage RNA polymerase (for example T7, T3, or Sp6 polymerase), of an appropriate template such as a recombinant plasmid containing a phage promoter and the target sequence cloned in the antisense orientation. The *in vitro* transcript is typically labeled by incorporation of a radiolabeled ribonucleotide and the protected probe is typically detected by polyacrylamide gel electrophoresis and autoradiography. The conventional RPA has certain features in common with the present invention, namely the use of a probe containing ribonucleotides, and post-hybridization treatment with RNase to eliminate probe that is not associated with target RNA. However, the RPA differs from the current invention by the use of a labeled probe which is detected directly, whereas in the current invention the probe is unlabeled and is not itself detected, rather the amplification products derived from the protected probe are detected, which provides for a more sensitive assay.

There are other probe amplification methods such as the cycling probe cleavage detection method. Examples of this can be found in U.S. Patent Nos. 4,876,187, 5,011,769, and 5,660,988. These methods generally involve the use of chimeric DNA-RNA-DNA probe in conjunction with RNase H, where hybridization of target DNA creates substrate for RNase H or exo III cleavage. Cleavage of the probe allows the probe fragments to dissociate from the target, allowing subsequent cycles of probe binding to the target, being cleaved and dissociating. The cleaved probe fragment can be detected by a variety of methods including tailing from its free 3' OH. The cycling probe reaction is relatively slow, limiting it to relatively abundant targets as compared to other amplification methods such as PCR™. Although the cycling probe cleavage reaction superficially resembles the current invention in that it utilizes a chimeric DNA-RNA-DNA probe, it differs in that the present invention the uncleaved chimeric probe is copied multiple times to amplify the signal from each copy of hybridized target. In cycling probe, the probe itself is cleaved multiple times for each copy of target. Another variation of the nuclease cleavage assay for detection of target nucleic acid is described in US Patent 4,876,187. This invention describes use of a chimeric oligonucleotide probe of the form DNA-RNA-DNA, which is hybridized to a sample potentially containing a target nucleic acid complementary to the central RNA portion of the probe, and is then treated with single-strand-specific ribonuclease under conditions effective to specifically cleave the unhybridized probe at the cleavable RNA linkage. Chimeric probe that is hybridized to target nucleic acid is double-stranded across the central RNA portion and thus spared from RNase cleavage. Uncleaved probe that survives RNase treatment is then detected, for example by association of a capture sequence at one end of the DNA-RNA-DNA chimeric probe with a detectable moiety at the opposite end of the DNA-RNA-DNA probe. Although the '187 invention has some aspects in common with certain embodiments of the present invention, it is distinguished from the current invention because the '187 invention is based on direct detection of the uncleaved chimeric probe, whereas in the current invention, the uncleaved probe is amplified and the product of the amplification reaction is then detected. The lack of a post-treatment amplification step results in lower sensitivity for detection of the target nucleic acid. Some other variations of nuclease protection techniques are also ultimately similarly limited in sensitivity because they do not involve probe amplification. U.S. Patents No. 6,232,066, 6,238,869, 6,458,533 and US Patent Application Pub. No. 2003/0096232 describe high throughput assay systems that involve a nuclease protection step using a probe, however, no amplification step is used and sensitivity is therefore reduced compared to the method of the current invention. U.S. Patent Application Pub. No. 2003/0170623 describes an array method using labeled microspheres and not probe amplification to increase detection.

Due to the inherent limitations of detection methods based only on hybridization of probes to unamplified targets, other methods have been developed for monitoring gene expression, in which the target mRNA is amplified to increase sensitivity and which are less laborious and time-consuming than traditional approaches. The most widely used method for target amplification and detection is the polymerase chain reaction (PCR™). PCR™ is disclosed in U.S. Patents No. 4,683,195 and 4,683,202. Briefly, two oligonucleotide primers that flank the DNA segment of the target sequence to be amplified are used to initiate sequential duplication of the target sequence. After heat denaturation, the primers hybridize to their complementary sequences on the opposite strands of the target and replication occurs enzymatically due to DNA synthesis from the two primers. Repetitive cycles of denaturation, primer annealing to target strands, and then synthesis are carried out so that there is replication of a complementary strand to each of the original strands per cycle. In turn, each of the product strands is capable of being hybridized to the primers. This results in an exponential amplification of the target nucleic acid followed by detection. However, there are a number of technical problems associated with PCR™. For example, problems can arise from the co-amplification of non-specific hybridization of primers to extraneous sequences along the target template. This non-specific amplification increases with each cycle. The current invention overcomes this limitation because the treatment used to eliminate the unhybridized chimeric probe (for example, treatment with ribonuclease) also eliminates all or most of the extraneous mRNAs that would otherwise be able to non-specifically hybridize to the primers and contribute to non-specific amplification. Another technical problem with PCR™ is that target sequences that differ in size and in the sequence of their PCR™ primer binding sites are amplified with different efficiencies. For this reason, the relative levels of multiple target sequences in a sample which are amplified in the same PCR™ reaction (*i.e*. which are amplified in a multiplex reaction) do not generally reflect the relative starting levels of the targets prior to amplification, making it impossible to deduce the relative abundance of the multiple unamplified targets in the sample. Other approaches employ target specific and universal primers at once in a multiplex reaction but require extensive data analysis (US 2003/0204322). The present invention overcomes this limitation by providing a method for amplifying a target using a probe having an amplification portion that is separate and distinct from the target-complementary portion of the probe, which permits identical primer binding sites to be used to produce amplicons of identical size from targets that differ in sequence. This allows the amplification portion of the probe to be optimized for amplification or multiplexing without having to worry about the constraint of the probe the hybridizing to the target. Thus, the chimeric probes of the present invention can be highly multiplexed by using sequences that are optimized for multiplex amplification. Once an amplification sequence has been developed, it can be used with any number of different targets in different assays.

Another technical problem with PCR™ is related to its inability to directly amplify very short DNA or cDNA targets, such as those obtained from highly degraded or crosslinked nucleic acid, frequently seen with formaldehyde fixed paraffin embedded tissue (FFPE), or targets such as miRNAs and small interfering RNAs (siRNAs), which are only - 21-23 bases in length. This limitation arises from the fact that the pair of oligonucleotide primers which flank the DNA segment of the target sequence to be amplified (which are used to initiate sequential copying of the target sequence) are each generally also - 20 bases in length, thus there is not sufficient space on the target to allow for annealing of the primers with an intervening sequence that can be amplified and detected. This problem cannot be solved by simply reducing the size of the primers (for example to - 5 bases in length), because they would then fail to hybridize stably to the target at temperatures needed for the enzymatic steps of RT-PCR and because they would lack sufficient specificity for hybridization to only the intended target RNA.

PCR™ can be used in conjunction with other enzymatic reactions. For real-time PCR™, a dual labeled fluorescent probe may be used that includes a reporter fluor and a quencher fluor. The dual labeled fluorescent probe hybridizes to the amplified product and the reporter fluor is cleaved from the probe by the intrinsic 5'-exonuclease activity of the thermostable polymerase, thereby generating detectable signal when the PCR™ product is produced during the amplification process. This is the principle behind the widely used TAQMAN® assay. Alternatively, signal can be generated by binding of a double-strand nucleic acid-specific dye to the amplified product as it accumulates during each cycle. See U.S. Patent Nos. 6,174,670; 5,723,591; 5,801,155 and 6,084,102. However, these methods have some of the same general problems as PCR™, in that they may be vulnerable to non-specific amplification and are unable to directly amplify very short DNA or cDNA targets, such as those obtained from miRNAs and small interfering RNAs.

Another technique for detection of nucleic acid targets uses multiplex amplifiable probe hybridization (Armour *et al.,* 2000 and U.S. Patent No. 6,706,480). In MAPH, the probes are hybridized to a sample, excess probe is washed away, and the hybridized probe is recovered and amplified by PCR™. The different probes are flanked by common primer binding sites so the whole collection of probes can be amplified together by PCR™. A variant of this procedure is the multiplex ligation-dependent probe amplification (MLPA) technique Schouten *et al.,* 2002). In this method pairs of probes, in the presence of the specific nucleic acid target, are ligated together to form molecules competent to be amplified by PCR™. The ligation reaction is dependent on the presence of the nucleic acid target. This should result in lower levels of background signal that seen with MAPH. Another variant is molecular inversion probes (M1P) (Hardenbol *et a*/*.,* 2003). This method involves the use of probes that cannot be amplified unless there is an enzymatic "gap fill" and ligation process that occurs after hybridization to a target sequence. This circularizes the probe, linking it to the target nucleic acid, allowing for very stringent washing. The probes are then released by cleavage of the probe at a uracil residue not located in the target specific portion of the probe. The MIP method differs in several respects from the current invention. The MIP method creates an amplification competent molecule after gap fill and ligation in the presence of target. In the present invention, a chimeric probe hybridized to target is protected from inactivation, excess probe is cleaved by nuclease. The cleavage step in MIP probes is not target dependent and merely serves to open up the circularized MIP probe. Other methods for detection of nucleic acid targets, including ligase chain reaction and "padlock probes", are based on target-dependent ligation of contiguous oligonucleotides. Unlike the present invention, ligation-based methods do not use a probe modifying agent (for example, a nuclease cleavage step) to eliminate unhybridized probe prior to the amplification step. There remains a need for improved methods for detecting, identifying, and/or quantifying a specific nucleic acid target in a sample, particularly short nucleic acid sequences and/or nucleic acids whose concentrations are relatively low.

### SUMMARY OF THE INVENTION

The present invention is based on protocols designed to overcome some of the limitations of the nucleic acid detection techniques discussed above. The present invention combines aspects of a nuclease protection assay and an amplification based assay.

In general, the present invention involves use of a probe called a "HARP probe." HARP stands for Hybridization Amplification RNase Protection. The HARP probe is comprised of at least 3 regions: a Target Hybridization Region, an Amplification Region, and a Probe Inactivation Region. In preferred embodiments the HARP probe also contains one or more Detection Region(s).

In some embodiments, the invention relates to methods for amplifying a target nucleic acid sequence comprising: contacting, under hybridization conditions, a nucleic acid comprising the target nucleic acid sequence with a HARP probe; exposing the HARP probe to one or more modifying agents that renders the HARP probe non-amplifiable when there is no hybridization between the HARP probe and the target nucleic acid sequence; and amplifying the sequence complementary to at least the Target Hybridization Region of the HARP probe using one or more Amplification Regions; wherein all or part of the HARP probe is amplified. In certain embodiments, methods include at least a hybridization step, an inactivation step, and an amplification step, in that particular order.

In some embodiments, methods for amplifying a target nucleic acid sequence involve, but are not limited to: a) contacting, under hybridization conditions, a nucleic acid comprising the target nucleic acid sequence with a HARP probe comprising i) a Target Hybridization Region that contains an Probe Inactivation Region and ii) at least one Amplification Region; b) exposing the HARP probe to one or more inactivating agents that modifies one or more residues in the Probe Inactivation Region of the probe if not hybridized to the target nucleic acid sequence; c) then amplifying the HARP probe that remains intact after treatment with the inactivating agent. In terms of the invention, the inactivating agent acts to "inactivate" unhybridized probe by eliminating or significantly reducing the ability of the unhybridized HARP probe to be amplified. In many embodiments, this inactivation will involve cleavage of the unhybridized probe.

In some specific embodiments, the HARP probe comprises an Amplification Region that comprises a promoter sequence, for example but not limited to a T3, T7, or SP6 promoter sequence. In additional embodiments, the HARP probe comprises at least two Amplification Regions for hybridization of amplification primers to permit amplification using PCR™. In some specific embodiments, the Target Hybridization Region is between 1 and 100 residues in length, more specifically between 1 and 50 residues in length, and even more specifically, between 10 and 30 residues in length.

The HARP probe may be chimeric, as described detail below. For example, the Target Hybridization Region may be chimeric. In some embodiments, a chimeric HARP probe comprises at least one ribonucleotide and contiguous deoxyribonucleotides. Additionally, a chimeric HARP probe may comprise an RNA portion and at least one DNA portion. In such case, the Target Hybridization Region may comprise all or part of the RNA portion. Some chimeric HARP probes comprise only one RNA portion and only one DNA portion, while others comprise at least one RNA portion and at least one DNA portion. In some cases, the Target Hybridization Region comprises both RNA and DNA. In some preferred embodiments, the chimeric HARP probe has a DNA portion followed by the RNA portion or an RNA portion followed by a DNA portion.

In some embodiments the HARP probe comprises a DNA portion that comprises at least one Amplification Region, and in even more specific embodiments, that DNA portion further comprises part of the Target Hybridization Region. In some preferred embodiments, the Amplification Region includes a promoter sequence that permits synthesis of multiple copies of the HARP probe using a suitable polymerase. Some embodiments involve a chimeric HARP probe that comprises two DNA portions. For example, the chimeric HARP probe may comprise a DNA portion, then an RNA portion, then a DNA portion. The chimeric HARP probe may comprise a DNA portion and a DNA portion comprising nucleotide analogs. In some specific embodiments, the DNA portion comprising nucleotide analogs is resistant to modification with the modifying agent(s). In some preferred embodiments, the DNA portion comprising nucleotide analogs is susceptible to modification with the modifying agent(s) when it is not hybridized. In some embodiments, the HARP probe is comprised of an RNA portion that fully complements all or part of the target nucleic acid sequence.

In some cases, the HARP probe comprises a second DNA region that includes a second Amplification Region. In such cases, the first and second DNA portions may flank the RNA region.

In some embodiments, the Amplification Region is adapted for use in exponential amplification. In some cases , the Amplification Region comprises a promoter sequence. In the practice of some embodiments, the HARP probe is amplified using the promoter sequence for *in vitro* transcription. The HARP probe can be amplified using one or more PCR™ reactions. The HARP probe can amplified in a buffer comprising KCI.

In many embodiments, the modifying agent modifies the HARP probe by cleaving it. For example, the modifying agent may enzymatically cleave the HARP probe. Preferred modifying agents include nucleases, including but nor limited to, S 1 nuclease, RNase A, RNase T1, and RNase 1. In other embodiments, the modifying agent is carbodiimide, osmium tetroxide, hydroxylamine, hydrazine, diethylpyrocarbonate, methylene blue, formic acid, potassium permanganate, and sodium hydroxide; and the method further comprising contacting the modified HARP probe with a cleaving agent. In some embodiments, the cleaving agent is piperidine.

In some preferred embodiments, the modifying agent is inactivated prior to amplification of the HARP probe. For example, a proteinase can be used to inactivate the modifying agent.

In some embodiments, the HARP probe is between 30 and 500 residues in length, more specifically between 50 and 100 residues in length.

In many embodiments, the methods of the invention further comprise detecting the amplified HARP probes. More specific embodiments comprise quantitating the amplified products of the HARP probes.

While the target nucleic acid can be any form of nucleic acid including RNA, DNA, or PNA, in some preferred embodiments, the target nucleic acid sequence is a ribonucleotide sequence, for example but not limited to, mRNA, rRNA, tRNA, miRNA, or siRNA. The target nucleic acid can also be derived from coding or noncoding regions of viral RNA or bacterial RNA.

Some specific embodiments relate to methods for amplifying one or more target nucleic acid sequences comprising: mixing, under hybridization conditions, at least a first nucleic acid molecule comprising the first target nucleic acid sequence with one or more HARP probes to form a hybridization mixture, wherein the HARP probe comprises a Target Hybridization Region and at least one Amplification Region; exposing the HARP probe(s) to one or more modifying agents that modifies modifiable residues in the Target Hybridization Region of the HARP probe(s) if not hybridized to the target nucleic acid; and amplifying the HARP probe(s), wherein the portion of the HARP probe complementary to the first nucleic acid sequence is amplified along with other sequences in the HARP probe .In some embodiments, the hybridization mixture comprises a second HARP probe for the first target nucleic acid sequence, wherein the second HARP probe comprises a Target Hybridization Region and at least one Amplification Region. The hybridization mixture may comprise a second HARP probe for a second target nucleic acid sequence in the first nucleic acid molecule, wherein the second HARP probe comprises a Target Hybridization Region and at least one Amplification Region. Some embodiments relate to assaying one or more target nucleic acid sequences comprising hybridizing one or more other target nucleic acid sequences and additional HARP probes for each of the other nucleic acid sequences. In such embodiments, the HARP probes for the nucleic acids may comprise one or two Amplification Regions that is/are distinct from a Target Hybridization Region. In more specific embodiments, the HARP probes for the nucleic acids can have the same Amplification Regions.

Other specific embodiments of the invention relate to methods for amplifying a target nucleic acid sequence comprising: contacting, under hybridization conditions, the target nucleic acid sequence with a chimeric HARP probe, wherein the chimeric HARP probe comprises: i) a Target Hybridization Region that is comprised of DNA nucleotides and of a Probe Inactivation Region composed of RNA nucleotides and ii) at least one Amplification Region, which is composed of DNA; exposing the chimeric HARP probe to one or more RNases that cleaves RNA comprising the Probe Inactivation Region in the Target Hybridization Region of the HARP probe if not hybridized to the target nucleic acid sequence; and amplifying at least the hybridization sequence of the HARP probe to create an amplified product. These methods may further comprise detecting and/or quantitating the amplified product. In some preferred embodiments, the chimeric HARP probe has at least two amplification domains. In some embodiments, the chimeric HARP probe has a DNA portion, followed by an RNA portion, followed by a second DNA portion. The chimeric HARP probe can have at least one Amplification Region composed of RNA. The chimeric HARP probe of such embodiments can have at least two Amplification Regions, which can be used to hybridize with a pair of PCR™ primers. In some embodiments, the chimeric HARP probe further comprises an additional DNA region comprising a Detection Region that comprises a detection probe binding site, which can be either a universal or target-specific detection probe binding site.

Further specific embodiments comprise methods for amplifying a target nucleic acid sequence comprising: contacting, under hybridization conditions, the target nucleic acid sequence with a chimeric HARP probe having one DNA segment followed by an RNA segment, wherein the DNA segment comprises at least a first Amplification Region and part of a Target Hybridization Region and the RNA segment comprises the remainder of the Target Hybridization Region and a Probe Inactivation Region and a second Amplification Region; exposing the chimeric HARP probe to one or more RNases that cleaves RNA in the Hybridization/Probe Inactivation/second Amplification Region of the HARP probe if not hybridized to the target nucleic acid sequence; and amplifying the hybridization sequence of the HARP probe to create an amplified product. The term "segment" as used in this context herein connotes a discrete region of contiguous nucleotides, which may be distinguished from an adjoining region. Further specific embodiments comprise the method similar to that described above, wherein the chimeric HARP probe comprises one RNA segment followed by one DNA segment, wherein the DNA segment comprises at least a first Amplification Region and part of a Target Hybridization Region and the RNA segment comprises the remainder of the Target Hybridization Region and a Probe Inactivation Region and a second Amplification Region; exposing the chimeric HARP probe to one or more RNases that cleaves RNA in the Hybridization/Probe Inactivation/second Amplification Region of the HARP probe if not hybridized to the target nucleic acid sequence; and amplifying the hybridization sequence of the HARP probe to create an amplified product.

In some embodiments, the invention relates to multiplex HARP assays. The invention allows for a significant improvement over prior multiplex methods for the amplification of specific targets, which typically use a different primer pair for each target sequence to be amplified. The present invention permits multiplex amplification of many distinct target nucleic acids using a common pair of PCR™ primers, which avoids the problems cited above. This is made possible since the binding sites for the PCR™ primers are located in the external regions of the HARP probe, rather than within the target-complementary region itself. In prior multiplex assays, which employ different primer pairs for each target sequences, differences in annealing efficiency of different primer pairs result in a strong bias in the amplification of the different amplicons thereby strongly reducing the fidelity of a quantitative multiplex assay. Furthermore the presence of a large number of different primers results in a strongly increased risk of primer dimer formation diminishing the possibility of reproducible amplifying small amounts of target nucleic acids.

Some embodiments of the invention relate to the ability of HARP probes and analyses to particularly function in the context of short RNAs. Short RNAs, such as miRNAs, siRNAs, and degraded RNA, cannot be detected by conventional reverse transcription-PCR methods (because they are shorter than the oligonucleotide primers used for PCR™ amplification), and so detection of miRNAs has relied on relatively insensitive techniques such as Northern blots and RNase protection assays. These assays may not be able to detect low-abundance miRNAs in RNA samples of reasonably high mass amount (*i.e*. microgram amounts), and are almost certainly insufficient for detecting even highly abundant miRNAs in low-mass-amount samples (*i.e*., picogram - nanogram amounts of RNA) such as those obtained from microdissected tissue. Candidate miRNAs have been proposed based on computational methods and confirmed in some cases by cloning and sequencing size-fractionated cellular RNA. Such sequenced-based studies, which are costly and laborious, yield a limited amount of information about miRNA expression patterns and lack the sensitivity needed to detect low-abundance miRNAs. In order to better elucidate the biological function of miRNAs, more sensitive methods are needed detect and quantify miRNAs.

Some embodiments of the HARP assay relate to the specific design of the First-strand primer, which is the oligonucleotide used to prime synthesis of the complementary strand of the HARP probe; said synthesis comprises the initial step of the amplification of the HARP probe. One advantageous design for the First-strand primer is that in which the 3' end of said primer traverses the RNA segment comprising the Probe Inactivation Region of the HARP probe. Such design avoids the need to use a reverse transcriptase enzyme or DNA polymerase enzyme with reverse transcriptase activity, for synthesis of the HARP-complementary strand, since the template for the initial nucleotide and all subsequent nucleotides incorporated into the complementary strand are DNA bases instead of RNA bases. In many embodiments of the HARP assay, the First-strand primer has a sequence which is different from that of the Reverse primer in the PCR™ amplification step. The First-strand primer and the Reverse primer are however related in that the product generated by the Forward PCR™ primer and the First-strand primer are able to hybridize to the Reverse primer, in order to permit amplification by subsequent cycles of PCR™. A consequence of this is that the sequence comprising the 5' side of the First-strand primer is completely or substantially identical to the sequence which occurs at least at the 3' side of the Reverse primer used for PCR™; that is, the 5' end of the First-strand primer overlaps the 3' end of the Reverse PCR™ primer. Particularly advantageous is a design in which the 5' side of the First-strand primer is entirely identical to the Reverse primer. The sequence of the Reverse primer in such cases is a subset of the sequence of the First-strand primer. The advantage of using a Reverse primer which is different from the First-strand primer is that a common Reverse primer (also known as a Universal Reverse Primer) may then be used for amplification of multiple HARP probes designed to detect distinct targets; this will minimize the problems of bias in relative levels of amplified products introduced during amplification, and of unwanted primer interactions.

The embodiments discussed in further detail in the Detailed Description Section below provide additional information regarding the compositions and methods of the invention.

Any embodiment discussed with respect to compositions and methods of the invention, as well as any embodiment in the Examples, is specifically contemplated as being part of a kit.

Unless otherwise specified, the order of nucleotides or regions in a probe is recited from 5' to 3'.

It is contemplated that any method or composition described herein can be implemented with respect to any other method or composition described herein.

Throughout this application, the term "about" is used to indicate that a value includes the standard deviation of error for the device or method being employed to determine the value.

The use of the word "a" or "an" when used in conjunction with the term "comprising" in the claims and/or the specification may mean "one," but it is also consistent with the meaning of "one or more," "at least one," and "one or more than one."

It is specifically contemplated that any embodiments described in the Examples section are included as an embodiment of the invention.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
- **FIG.** 1: HARP assay for miRNA expression profiling allowing for quantitative expression analysis using the TAQMAN system.
- **FIG. 2**: HARP assay for miRNA expression profiling allowing for quantitative expression analysis using the TAQMAN system and employing conversion of HARP probe to cDNA complement by reverse transcription with an appropriate reverse transcriptase enzyme prior to quantitative PCR™.
- **FIG. 3**: HARP assay for miRNA expression profiling allowing for quantitative expression analysis using a fluorescent labeled bead system.
- **FIG. 4**: HARP assay for miRNA expression profiling allowing for quantitative expression analysis using a fluorescent labeled bead system and employing conversion of HARP probe to cDNA complement by reverse transcription with an appropriate reverse transcriptase enzyme prior to quantitative PCR™_{.}

### DETAILED DESCRIPTION OF' INVENTION

The present invention is directed to compositions and methods relating to amplifying one or more probes for one or more target nucleic acid sequences for the purpose of detecting and/or quantifying the target nucleic acid sequence. These methods can be implemented to detect, identify, quantitate, and/or distinguish i) a particular nucleic acid sequence in a particular mixture of nucleic acid molecules; ii) multiple nucleic acid sequences in the same mixture of nucleic acid molecules; iii) multiple nucleic acid sequences in multiple mixtures of nucleic acid molecules; and iv) differences in sequence between or among cognate nucleic acid sequences, such as mutations or single nucleotide polymorphisms. The methods generally involve a hybridization step using a HARP probe; a modification step to distinguish hybridized HARP probe from an unhybridized HARP probe; and an amplification step in which the probe is amplified. Though not intended to be limiting, ways of implementing these steps and reagents that can be used for them are discussed below.

### I. Nucleic Acids and Preparation Thereof.

The present invention concerns probes that can be used to detect, identify, and/or quantitate a target nucleic acid sequence by virtue of the chemical bonding (also known as hybridization) that occurs between complementary sequences. The probes are composed of nucleic acids (or nucleotide analogs), though non-nucleic acid components may be included.

### A. Nucleic Acids

The term "nucleic acid" is well known in the art. A "nucleic acid" as used herein will generally refer to a molecule (one or more strands) of DNA, RNA or a derivative or analog thereof, comprising a nucleobase. A nucleobase includes, for example, a naturally occurring purine or pyrimidine base found in DNA (*e.g*., an adenine "A," a guanine "G," a thymine "T" or a cytosine "C") or RNA (*e.g*., an A, a G, an uracil "U" or a C). The term "nucleic acid" encompass the terms "oligonucleotide" and "polynucleotide," each as a subgenus of the term "nucleic acid." Generally probes of the invention are single stranded, though they can be partially double stranded.

The term "recombinant" may be used and this generally refers to a molecule that has been manipulated *in vitro* or that is the replicated or expressed product of such a molecule.

Nucleic acid molecules of the invention can be composed of any residues discussed herein, including those discussed in more detail below.

As used herein a "nucleobase" refers to a heterocyclic base, such as for example a naturally occurring nucleobase (*i.e*., an A, T, G, C or U) found in at least one naturally occurring nucleic acid (*i.e*., DNA and RNA), and naturally or non-naturally occurring derivative(s) and analogs of such a nucleobase. A nucleobase generally can form one or more hydrogen bonds ("anneal" or "hybridize") with at least one naturally occurring nucleobase in manner that may substitute for naturally occurring nucleobase pairing (*e.g*., the hydrogen bonding between A and T, G and C, and A and U).

"Purine" and/or "pyrimidine" nucleobase(s) encompass naturally occurring purine and/or pyrimidine nucleobases and also derivative(s) and analog(s) thereof, including but not limited to, those a purine or pyrimidine substituted by one or more of an alkyl, caboxyalkyl, amino, hydroxyl, halogen (*i.e*., fluoro, chloro, bromo, or iodo), thiol or alkylthiol moiety. Preferred alkyl (*e.g*., alkyl, caboxyalkyl, etc.) moieties comprise of from about 1, about 2, about 3, about 4, about 5, to about 6 carbon atoms.

A nucleobase may be comprised in a nucleoside or nucleotide, using any chemical or natural synthesis method described herein or known to one of ordinary skill in the art. Such nucleobase may be labeled or it may be part of a molecule that is labeled and contains the nucleobase.

As used herein, a "nucleoside" refers to an individual chemical unit comprising a nucleobase covalently attached to a nucleobase linker moiety. A non-limiting example of a "nucleobase linker moiety" is a sugar comprising 5-carbon atoms (*i.e*., a "5-carbon sugar"), including but not limited to a deoxyribose, a ribose, an arabinose, or a derivative or an analog of a 5-carbon sugar. Non-limiting examples of a derivative or an analog of a 5-carbon sugar include a 2'-fluoro-2'-deoxyribose or a carbocyclic sugar where a carbon is substituted for an oxygen atom in the sugar ring.

Different types of covalent attachment(s) of a nucleobase to a nucleobase linker moiety are known in the art. By way of non-limiting example, a nucleoside comprising a purine (*i.e*., A or G) or a 7-deazapurine nucleobase typically covalently attaches the 9 position of a purine or a 7-deazapurine to the 1'-position of a 5-carbon sugar. In another non-limiting example, a nucleoside comprising a pyrimidine nucleobase (*i.e*., C, T or U) typically covalently attaches a 1 position of a pyrimidine to a 1'-position of a 5-carbon sugar (Kornberg and Baker, 1992).

It is contemplated that embodiments of the invention could be carried out using a probe containing modified bases instead of the typical DNA-RNA-DNA form of the probe, or consisting of only DNA bases. In these formats, the central region would still be complementary to the target, and the flanking regions used for amplifying the protected probe would be resistant to the post-hybridization treatment (*i.e*., nuclease digestion or chemical cleavage) used to eliminate unhybridized probe. One way to make the flanking regions resistant to cleavage would be to synthesize them with chemical modifications such as 2'-o-methyl modifications that confer resistance to enzymatic digestion. In this version of the assay (using a DNA probe), the central target-complementary region of the probe would be synthesized without modifications that confer stability to the post-hybridization HARP probe inactivation treatment. Only probes that were hybridized to their complementary RNA or DNA targets would be resistant to the post-hybridization treatment used to prevent amplification of probes not associated with target. Another strategy would be to use DNA-only probes and include a molar excess of oligonucleotides complementary to the flanking Amplification Regions and Detection Regions of the probes in the hybridization reaction; the complementary oligos would hybridize to these flanking regions, rendering them double-stranded and therefore resistant to cleavage by single-strand-specific nucleases such as S1 nuclease. Another example of a non-standard HARP probe is to make the flanking region from a non-cleavable nucleotide analog, for example from peptide nucleic acid (PNA). After hybridization, the reaction would be treated with a single-strand-specific DNase such as S1 nuclease and probes that were not hybridized to their target DNA or RNA would be cleaved, while the probes that survive the nuclease digestion step would be amplified and detected.

Thus, a nucleic acid may comprise, or be composed entirely of, a derivative or analog of a nucleobase, a nucleobase linker moiety and/or backbone moiety that may be present in a naturally occurring nucleic acid. RNA with nucleic acid analogs may also be used in chimeric constructs according to methods of the invention. As used herein a "derivative" refers to a chemically modified or altered form of a naturally occurring molecule, while the terms "mimic" or "analog" refer to a molecule that may or may not structurally resemble a naturally occurring molecule or moiety, but possesses similar functions. As used herein, a "moiety" generally refers to a smaller chemical or molecular component of a larger chemical or molecular structure. Nucleobase, nucleoside and nucleotide analogs or derivatives are well known in the art, and have been described (see for example, Scheit, 1980).

Additional teachings for nucleoside analogs and nucleic acid analogs are U.S. Patent 5,728,525, which describes nucleoside analogs that are end-labeled; U.S. Patent 5,637,683, 6,251,666 (L-nucleotide substitutions), and 5,480,980 (7-deaza-2'deoxyguanosine nucleotides and nucleic acid analogs thereof).

Additionally nucleic acid analogs may include those that can be labeled with a dye, including a fluorescent dye, or with a molecule such as biotin. Labeled nucleotides are readily available; they can be acquired commercially or they can be synthesized by reactions know to those of skill in the art.

### B. Preparation of Nucleic Acids

A nucleic acid may be made by any technique known to one of ordinary skill in the art, such as for example, chemical synthesis, enzymatic production or biological production. It is specifically contemplated that HARP probes of the invention are chemically synthesized. It is contemplated that a biological sample may be treated in a way so as to enhance the recovery of small RNA molecules such as miRNA. U.S. Patent Application Serial No. 10/667,126 describes such methods. Generally, methods involve lysing cells with a solution having guanidinium and a detergent.

### II. HARP Probes and Design Thereof

In general, a HARP probe is comprised of at least 3 regions: a Target Hybridization Region, an Amplification Region, and a Probe Inactivation Region. In preferred embodiments the HARP probe also contains one or more Detection Region(s).

More specifically, the HARP probes comprise a Target Hybridization Region, which is complementary to all or part of a target nucleic acid sequence, and an Amplification Region, which allows all or part of the probe to be amplified. Furthermore, because hybridized probe is distinguishable from unhybridized probe using an agent that inactivates the probe based on this difference, the probe contains a Probe Inactivation Region with one or more modifiable residues that are contained in the Target Hybridization Region. A particular benefit of the HARP probe is that it can serve as a nucleic acid template for amplification, said template being longer than the original endogenous targeted nucleic acid.

Because HARP probes are nucleic acids, they, by definition, have 5' ends and 3' ends, as will be understood by those of skill in the art. Additionally, in the context of the invention, the various regions of a given HARP probe have a "5' end" and a "3' end", even though such an "end" of a region may not be at the physical end of the nucleic acid forming the HARP probe. Rather, in the context of HARP probe regions, the 5' end of a given region is the end nucleic acid base of the region at or closest to the physical 5' end of the probe and the 3' end of a given region is the end nucleic acid base at or closest to the physical 3' end of the probe.

The HARP probe is, in many embodiments, chimeric, meaning that it is comprised of bases that differ in their chemical properties, for example in their susceptibility to cleavage with nucleases such as RNase or DNase. For example, the HARP probe may be chimeric in that it is comprised of both DNA and RNA, or it may comprise modified and unmodified DNA or modified and unmodified RNA, or combinations of modified and unmodified DNA and RNA. Potential modifications include analogs of deoxyribonucleotides and of ribonucleotides and also peptide nucleic acids (PNAs) or other non-natural nucleic acid analogs. In particular embodiments, the probe is recombinant or synthetic. Moreover, the length of the probe can be, be at least, or be at most 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500 residues or more, or any range derivable therein. In certain embodiments the probe is between 30 and 500 residues in length, particularly between 50 and 100 residues in length.

In some embodiments the HARP probe is attached to a solid support or it contains a biotin or other moiety that will allow the HARP probe to be attached to a solid support at some point during the assay. For example, biotinylated HARP probes could be captured after the hybridization step and then washed, cleaved, washed to remove the RNase and then added to an *in vitro* transcription reaction.

Features of the various regions of the HARP probe are described in more detail below.

### A. Target Hybridization Regions

The term "Target Hybridization Region" refers to a region of nucleotides (or nucleotide analogs) within the probe that are complementary to a sequence contained within the target nucleic acid. The Target Hybridization Region is thus comprised of a segment that is complementary to all or part of the targeted nucleic acid.

Target Hybridization Regions are designed to hybridize to a given nucleic acid sequence, where the sequence and the HARP probe are place "under hybridization conditions," meaning that the nucleic acids (including modified nucleic acids) are placed under conditions that promote intermolecular formation of Watson/Crick base pairs by hydrogen bonding between complementary sequences. Such conditions generally involve specific temperature and salt concentrations. Other parameters may influence the conditions, such as nucleic acid concentration-including the concentration of the complementary sequences, presence of a detergent, and presence of isostabilizing agents. Conditions that promote hybridization between a nucleic acid probe and a target nucleic acid are well known to those of skill in the art.

In general, a Target Hybridization Region comprises at least five residues complementary to a targeted nucleic acid molecule and at least one residue within the Target Hybridization Region that is susceptible to modification when in its unhybridized single-stranded state. By definition the Target Hybridization Region is complementary to the target; see preceding paragraph. It is contemplated that the Target Hybridization Region includes, includes at least, or includes at most 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, S5, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500 or more contiguous nucleotides (or nucleotide analogs), or any range derivable therein.

A HARP probe may contain multiple Target Hybridization Regions. In some embodiments, the multiple regions are complementary to different segments on the same targeted nucleic acid, for example different regions of a single mRNA. In other embodiments, the multiple regions are complementary to distinct targeted nucleic acids. When a HARP probe contains multiple Target Hybridization Regions, they may be contiguous or they may be separated by nucleotides that are not complementary to targeted nucleic acids. Methods of the invention may involve a probe with 1, 2, 3, 4, 5 or more Target Hybridization Regions.

Moreover, it is contemplated that the HARP probe used in methods of the invention can have residues that can be modified when exposed to a modifying agent and when not hybridized to the target sequence. These modifiable residues will be in the Target Hybridization Region of the HARP probe, and they comprise the Probe Inactivation Region.. In certain embodiments, the number of modifiable residues in the target probe is, is at least, or is at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500 residues or more (or any range derivable therein), which may or may not be contiguous. In preferred embodiments, the modification is accomplished though enzymatic cleavage, in which case the cleavage step inactivates the probe by eliminating or significantly reducing the ability of the HARP probe to be amplified.

In specific embodiments; the Target Hybridization Region of the HARP probe includes RNA. The Target Hybridization Region may consist entirely of RNA, while in other embodiments, only part of the Target Hybridization Region is RNA. There may be, be at least, or be at most 1, 2,... 500 or more ribonucleotides (or any range derivable therein) in the Target Hybridization Region. The ribonucleotides may or may not be contiguous. Furthermore, in preferred embodiments the Target Hybridization Region also contains DNA nucleotides. Regions of RNA or DNA may be referred to as an "RNA segment" or a "DNA segment," respectively. In these embodiments, the Target Hybridization Region has, has at least, or has at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 5, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500 or more deoxyribonucleotides (or any range derivable therein). A consequence of having DNA segments in the Target Hybridization Region is that the DNA bases are in some cases resistant to modification by the agents, for example RNases, that are used to modify (i.e. cleave) unhybridized probes to render them incapable of being amplified. This may be advantageous to prevent cleavage of hybridized HARP probes in regions of local denaturation between probe and target, for example in A+U-rich regions. Some miRNAs that may be target nucleic acids for the HARP assay are comprised of segments containing only A and U residues, for example miR-16 contains a segment of 8 bases comprised entirely of A and U residues. Such segments are prone to transient local denaturation when hybridized to their complementary sequences, due to the fact that A-U base pairs are held together with only 2 hydrogen bonds, as opposed to the more stable C-G base pairs, which are held together with 3 hydrogen bonds. A HARP probe containing only RNA bases in the Target Hybridization Region would therefore be susceptible to RNase cleavage in the A+U-rich segment when hybridized to its miR-16 target nucleic acid. Such non-specific cleavage of the hybridized HARP probe would prevent the probe from being amplified and thus reduce sensitivity of the assay. This problem can be prevented by designing the HARP probe to contain DNA bases, 2-o-methyl RNA or other non-cleavable bases instead of RNA bases, in that part of the Target Hybridization Region which is subject to local denaturation.

### B. Amplification Regions

The Amplification Region comprises one or more segments that can be used to amplify all or part of the HARP probe. The term "Amplification Region" refers to a region of nucleotides (or nucleotide analogs) that can be used to amplify the probe sequence. As is discussed in further detail below, amplification can be exponential, as with PCR™, or linear, as with *in vitro* transcription. Amplification can be carried out using many different nucleic acid amplification procedures.

Moreover, the Amplification Region of each HARP probe can include, include at least, or include at most 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500 or more contiguous nucleotides (or nucleotide analogs), or any range derivable therein. In general the HARP probe is designed such that the PCR™ primers and TAQMAN® probe, used respectively to amplify and detect it, will be have minimal tendency to form homodimers, "hairpins" (i.e. regions of internal secondary structure), and heterodimers.

The Amplification Region can be comprised of 2 or more noncontiguous segments, for example Forward and Reverse primer binding sites, to allow amplification of the HARP probe by PCR™. Alternatively, the Amplification Region can comprise a promoter sequence to permit amplification by a phage polymerase.

In a specific embodiment, where the HARP probe includes the following contiguous segments: DNA-RNA-DNA, it is contemplated that the first DNA segment can contain at least one Amplification Region, while the second DNA segment contains another Amplification Region. Particularly suitable is a pair of PCR™ primer binding sites, one located in each Amplification Region, such as a forward primer binding site in the first DNA segment and a reverse primer binding site in the second DNA segment. It is contemplated that all or part of the Target Hybridization Region can be flanked by Amplification Regions that completely or partially comprise PCR™ primer binding sites.

In some embodiments of the invention, amplification is achieved by a PCR™ or *in vitro* transcription reaction. As is discussed in further detail below, amplification can be exponential, as with PCR™, or linear, as with *in vitro* transcription. Amplification can be carried out using many different nucleic acid amplification procedures. Thus in some embodiments of the invention, amplification is achieved by a PCR™ or *in vitro* transcription reaction. It is contemplated that a HARP probe may contain more than one Amplification Region, for example a pair of PCR™ primer binding sites and a T7 phage promoter sequence, or two pairs of nested PCR™ primer binding sites. In the method of the present invention the HARP probe can be used for multiplexing, in which case multiple HARP probes having distinct Target Hybridization Regions can be included in a single hybridization reaction.

In some embodiments, portions of the Amplification Region, Target Hybridization Region, and Probe Inactivation Region overlap in the HARP probe. In some embodiments of the invention, the Amplification Region may comprise a promoter sequence (for example a T7 phage promoter) that is operatively associated with the Target Hybridization Region in the HARP probe, in order to permit amplification of the HARP probe by *in vitro* transcription with an appropriate polymerase such as T7 RNA polymerase. Any promoter sequence can be used, but those particularly contemplated are those routinely used in *in vitro* transcription reactions, such as a promoter selected from the group consisting of a T3, a T7, or a SP6 promoter.

In preferred embodiments, the HARP probe has a pair of Amplification Regions that can be used to hybridize to primers for PCR™ reactions, in order to amplify the HARP probe. An Amplification Region that is to be used as a primer-binding site for amplification by PCR™ may or may not have a sequence that is complementary to the targeted nucleic acid sequence, i.e. a sequence comprising a Target Hybridization Region. The Amplification Region may be co-extensive with the Target Hybridization Region. That is, the target-complementary sequence may partially or completely comprise the primer-binding site for one or both of the PCR™ primers used to amplify the HARP probe. The target-complementary site may also partially or completely comprise the binding site for an oligonucleotide used to prime synthesis of the complement of the HARP probe by Reverse Transcriptase enzymes.

### C. Inactivation Regions

The Probe Inactivation Region of the HARP probe contains nucleotides that can be modified to prevent amplification of the HARP probe when it is not hybridized to its target nucleic acid. In general, the Probe Inactivation Region comprises a subset of the nucleotides within the Target Hybridization Region. To reduce or prevent amplification of a HARP probe which is not hybridized to its target nucleic acid, and thus make amplification of the HARP probe dependent on the presence of the target nucleic acid, a post-hybridization probe inactivation step is carried out using an agent which is able to distinguish between a HARP probe that is hybridized to its targeted nucleic acid sequence and the corresponding unhybridized HARP probe. The agent is able to inactivate or modify unhybridized HARP probe such that it cannot be amplified. In some embodiments the agent can also be used to distinguish a HARP probe that is hybridized to a completely complementary target nucleic acid sequence from a HARP probe hybridized to a related target containing one or more basepair mismatches with the HARP probe, for the purpose of distinguishing between related target sequences. In the preferred embodiment, the Probe Inactivation Region of the HARP probe is comprised of nucleotides that can be cleaved by a cleaving agent when they are in their unhybridized, single-stranded state, and which are resistant to cleavage when they are hybridized to their target nucleic acid. The HARP probes also contain nucleotides that cannot be cleaved (non-cleavable portion), regardless of whether they are single-stranded or double-stranded. It is in this sense that the HARP probe is said to be chimeric; it contains nucleotides that are susceptible to inactivation (for example, inactivation by cleavage) when they are single-stranded, and it contains nucleotides that are not susceptible to inactivation when they are single-stranded. An example of this chimeric property is a HARP probe composed of DNA and RNA, where the cleaving agent is a ribonuclease, such as RNase A, RNase T1, or RNase 1, that cleaves single stranded RNA; the HARP probe will be susceptible to cleavage in the RNA portion of the molecule when it is not hybridized to a complementary sequence. Probes not cleaved can be amplified by any of a variety of methods such as PCR™, *in vitro* transcription or other amplification methods. For PCR™ amplification, the PCR™ primer binding sites can be located such that they completely or partially flank the RNA portion of the HARP probe.

### D. Detection Regions

The Detection Region of the HARP probe refers to a segment used to detect the amplified product. The use of various detection methods in the context of the HARP assay are discussed in further detail below.

Detection Regions may be distinct or may overlap the Amplification Region and/or the Target Hybridization Region of the HARP probe. In some cases, a Detection Region comprises a binding site for a dual-labeled fluorescent probe (TAQMAN probe), which can be used for detection of the amplified product of the HARP probe. The binding site for a dual-labeled fluorescent probe may be "universal", that is common to HARP probes having distinct Target Hybridization Regions, or the binding site for a dual-labeled fluorescent probe may have a different sequence in different HARP probes. Other types of distinct regions may be present in the HARP probe, to permit detection of the amplified product of the HARP probe using detection reagents such as molecular beacons, molecular scorpions, dual-labeled fluorescent probes with modifications such as minor groove binding modifications, black-hole quencher modifications, locked nucleic acid modifications, etc. The Detection Region is contemplated to be, be at least, or be at most 3, 4, 5, 6, 7, 8, 9, 10, 11, 12, 13, 14, 15, 16, 17, 18, 19, 20, 21, 22, 23, 24, 25, 26, 27, 28, 29, 30, or 50 residues in length, or any range derivable therein. HARP probes may also contain other types of distinct regions to permit specific association (for example by hybridization of complementary sequences) of the amplified product of the HARP probe with solid supports such as addressable beads (for example those used in Luminex technology) or such as glass slide or nylon membrane-based microarrays.

The terms "barcodes", "adapters", tags" and "zip codes" have all been used to describe sequences that are added to amplicons to allow detection of distinct nucleic acids in pools of nucleic acids These types of sequences are also examples of Detection Regions that may be incorporated into HARP probes. One preferred form of barcodes are hybridization barcodes. In this embodiment barcodes are chosen so as to allow hybridization to the complementary capture probes on a surface of an array. Barcodes serve as unique identifiers of the probe. In general, sets of barcodes and the corresponding capture probes are developed to minimize cross-hybridization with both each other and other components of the reaction mixtures, including the target sequences and sequences on the larger nucleic acid sequences outside of the target sequences (*e.g*. to sequences within genomic DNA). Other forms of barcodes are mass tags that can be separated using mass spectroscopy, electrophoretic tags that can be separated based on electrophoretic mobility, etc

### E. Variations of Chimeric HARP Probes

As discussed above, the present invention further includes HARP probes that are chimeric. There are a variety of manners in which functional chimeric HARP probes can be constructed. Several examples of these manners are discussed below, and those of skill will, in view of this specification, be able to determine additional embodiments.

One reason for having a chimeric HARP probe is to enable the probe to be modified in only part of the probe when it is not hybridized to the targeting sequence. The portion that can be modified will be chimeric with respect to the remainder of the probe. The probe may be made of different chemical structures, such as one structure that is modifiable by a particular modifying agent, and another structure that is not susceptible to modification by that particular modifying agent. In certain embodiments, the chimeric HARP probe comprises one or more ribonucleotides and deoxyribonucleotides.

In certain embodiments, the Target Hybridization Region of the HARP probe is chimeric. It is contemplated that a chimeric HARP probe may have, have at least, or have at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45_{,} 46, 47, 48, 49, 50, 1, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500 or more residues (or any range derivable therein) in the Target Hybridization Region that are chimeric with respect to the amplification domain. The nucleotides may or may not be contiguous. In methods of the invention, a modifying agent acts on the Probe Inactivation Region within the Target Hybridization Region and modifies residues that are susceptible to modification if they are not hybridized to the target nucleic acid sequence. It is particularly contemplated that the modifiable residues constitute the Probe Inactivation Region of the target hybridization domain and that this region is chimeric with respect to the Amplification and Detection Region(s).

In particular embodiments, the number of modifiable residues in the Target Hybridization Region is between 1 and 100, between 1 and 50, between 3 and 20, or between 3 and 15 residues, which may or may not be contiguous.

When the chimeric HARP probe comprises RNA and DNA, it is contemplated that the probe may have a ribonucleotide segment and at least one deoxyribonucleotide segment.

The judicious selection of DNA and RNA bases at particular positions within the Target Hybridization Region of a HARP probe can be used to avoid nonspecific RNase cleavage. Such is an embodiment of the invention and is illustrated with the following sequences:
miR-16 target RNA: 5'-ua gcag cacg uaaa uauu ggcg (SEQ. ID NO:1)

A HARP probe for miR-16 containing only RNA bases (shown in lower case) in the Target Hybridization Region is shown below. Note, the underlined bases in the Target Hybridization Region of this probe would be vulnerable to non-specific cleavage when hybridized to miR-16. 5'-(Forward Primer Site)-(TAQMAN probe site): cgcc aaua uuua cgug cugc ua- (Reverse Primer Site) (SEQ ID NO:2)

A HARP probe for miR-16 containing 8 DNA bases (shown in UPPER CASE) in Target Hybridization Region is shown below. Note that the underlined region of this probe cannot be cleaved by RNase since it is DNA; the probe is still subject to cleavage by RNase when it is not hybridized to the miR-16 target, since the segments of the Target Hybridization Region comprised of RNA (*i.e*., the Probe Inactivation Region) will be cleaved when single-stranded.

5'-(Forward Primer Site)-(TAQMAN probe site)- cgcc AATA TTTA cgug cugc ua- (Reverse Primer Site) (SEQ. ID NO:3)

In preferred embodiments, the HARP probe has one or more Amplification Region(s) comprised of DNA. It is contemplated that 1, 2, 3, 4, 5 or more Amplification Regions on a HARP probe may be composed of DNA partly or completely.

In another specific embodiment, a chimeric HARP probe includes one or more DNA segments followed by an RNA segment. In this format the RNA segment is not flanked by DNA segments, but is positioned on either the 5' or the 3' side of one or more DNA segments. Furthermore, in this format, one of the PCR™ primer-binding sites (*i.e*. Amplification Regions) of the HARP probe is composed of DNA and the other is composed of RNA. The primer binding site which is composed of RNA also serves as the Target Hybridization Region of the HARP probe. Moreover, the Target Hybridization Region may be composed entirely of RNA or a combination of RNA and DNA. In specific embodiments, the HARP probe also contains a Detection Region comprising a universal probe binding site (for example, for a dual-labeled fluorogenic probe such as a TAQMAN probe) which will generally be made of DNA. In some cases, the Target Hybridization Region is primarily composed of RNA with 1, 2, 3, 4, 5, 6, 7, 8, 9, 10, 11, 12, 13, 14, 15, 16, 17, 18, 19, 20, 21, 22, 23, 24, 25 or more nucleotides being DNA. It is contemplated that the DNA segment in the Target Hybridization Region may be contiguous with other DNA segments in the HARP probe, such as DNA comprising an Amplification Region or a Detection Region, such as a dual-labeled fluorogenic probe-binding site. Specifically contemplated is a forward or reverse PCR™ primer binding site (*i.e.* an Amplification Region) made completely or partially of RNA and which also comprises the Target Hybridization Region as well as the Probe Inactivation Region. Thus, in one embodiment, there is a DNA-RNA probe that has the following general structure: 5'-DNA segment (Amplification Region 1, *i.e*. forward PCR™ primer binding site); RNA segment (Target Hybridization Region, Probe Inactivation Region, and Amplification Region 2-PCR primer binding site). In another embodiment, the HARP probe also contains a DNA segment comprising a Detection Region, for example a binding site for a dual labeled probe (TAQMAN probe). Such a HARP probe may have the following general structure: 5'- DNA segment (Amplification Region 1, *i.e*. Forward PCR™ primer binding site); Detection Region (DNA segment comprising binding site for a dual labeled probe); Target Hybridization Region/Probe Inactivation Region/Amplification Region 2 (RNA segment comprising both the target-complementary sequence and the Reverse PCR™ primer binding site)-3'. In a slightly modified version of this format, the first (5'-most) several (~2-6) bases of the Target Hybridization Region/Amplification Region 2 are comprised of DNA instead of RNA; the advantage of having several DNA bases at this position is to prevent nonspecific RNase cleavage at the region of local denaturation that may occur at the internal end of the target/probe duplex; such nonspecific cleavage would remove the Reverse PCR™ primer binding site, thereby preventing amplification of the hybridized HARP probe. Nonspecific RNase cleavage that occurred at the external end of the Target Hybridization Region could probably be tolerated, since it would only shorten the Reverse PCR™ primer binding site by a few bases, and hence would probably not prevent amplification of the HARP probe.

Thus, in some specific embodiments, the invention relates to methods for detecting a target nucleic acid sequence using the HARP assay comprising: a) contacting, under hybridization conditions, the target nucleic acid sequence with a chimeric HARP probe, wherein the chimeric HARP probe comprises i) a Target Hybridization Region that is composed of RNA and DNA nucleotides and ii) at least one Amplification Region, which is composed of DNA; b) exposing the chimeric HARP probe to one or more RNases that cleave the Probe Inactivation Region within the Target Hybridization Region of the HARP probe if not hybridized to the target nucleic acid sequence; c) then amplifying the HARP probe; and, d) detecting the products of the amplified HARP probe.

Other ways in which the HARP probe may be chimeric are that it may contain central residues of RNA in its Target Hybridization Region, which are susceptible to modification by RNases such as RNase A, T1, and 1 when not double-stranded, and external regions of a non-modifiable RNA analog such as 2'-OMe RNA. Alternatively, the chimeric probe can contain a Target Hybridization Region with residues comprised of single stranded DNA that are susceptible to modification by single strand specific nuclease digestion, such as S1 nuclease, and Amplification Regions and Detection Regions comprised of double-stranded DNA, which are not modifiable by S1 nuclease: Moreover, DNA analogs can be used in a chimeric HARP probe construct. The nucleotide analog can be resistant to modification with the modifying agent(s). Any embodiments discussed with respect to a particular chimeric structure, such as a DNA-RNA construct, can be implemented with respect to any other chimeric structure, insofar as the modification step achieves a similar result-*i.e*., differentiates between HARP probes whose Target Hybridization Region is or is not hybridized to the target nucleic acid.

### F. Exemplary HARP Probes

A HARP probe design particularly contemplated is one that is DNA-RNA-DNA or DNA-RNA. Amplification of a protected HARP probe by PCR™ can be preceded by conversion of the protected chimeric probe to its complement containing only DNA bases by reverse transcription of the chimeric probe with enzymes such as MMLV-reverse transcriptase, AMV-RT, Tth, reverse transcriptases with reduced or absent RNase H activity, etc. Amplification of chimeric DNA-RNA-DNA HARP probes by PCR™ is generally more efficient when the probes are converted to their complementary sequences containing only DNA bases prior to the PCR™ step. Conversion of chimeric DNA-RNA-DNA HARP probes to their complementary sequences by reverse transcriptases is generally more efficient when the oligonucleotide primer used for reverse transcription (also called the "first strand primer") is designed such that its 3' end anneals within or immediately adjacent to the RNA region of a chimeric DNA-RNA-DNA HARP probe. This situation avoids the need for the reverse transcriptase to initiate synthesis using DNA bases as template. An alternative to the use of reverse transcriptase for efficient conversion of chimeric DNA-RNA-DNA HARP probes to their complementary DNA-only sequences prior to PCR™, is to use DNA polymerase enzymes (e.g., Taq polymerase) to extend a primer that is designed such that its 3' end extends beyond the RNA region of the chimeric DNA-RNA-DNA HARP probe. In this way, the need for DNA polymerase to use RNA bases as template is avoided. Use of RNA bases as template by DNA polymerases may be less efficient compared to use of DNA bases as template. By using a primer that is designed such that its 3' end extends beyond the RNA region of the chimeric DNA-RNA-DNA HARP probe, the need to include a reverse transcription step prior to PCR™ amplification is avoided. When using a primer that is designed such that its 3' end extends beyond the RNA region of the chimeric DNA-RNA-DNA HARP probe, it may be preferable to design the RNA region of the chimeric probe in such a way to maintain specificity for hybridization of the TAQMAN probe to the PCRTM-amplified product of the chimeric probe. This ensures that non-specific amplification products such as "primer dimer" will not be detected during the PCR™ step. In order to design the chimeric probe in a way to maintain specificity for hybridization of the TAQMAN probe to the PCR™-amplified product, it may be advantageous to design the HARP probe such that the RNA region is kept to a minimum in size (~ 1 to ~ 5 bases) and such that the RNA region is located completely or partially on the 3' side of the Detection Region in the HARP, for example on the 3' side of the binding site for the TAQMAN probe. In general, in order to use the strategy where a Reverse primer is designed such that its 3' end extends beyond the RNA region of the chimeric DNA-RNA-DNA HARP probe, the RNA region of the HARP probe should contain only a few bases of RNA and these bases should be positioned near the 3' end of the Target Hybridization Region of the HARP probe, generally within - 5 bases from the 3'end of the Target Hybridization Region.

The advantages of using a HARP probe comprising a DNA-RNA construct are that the RNA region can be relatively long, extending over a greater portion of the target, which provides a more effective substrate for RNase digestion, thereby potentially reducing or eliminating nonspecific amplification of unhybridized probes following the RNase treatment. Other advantages of the DNA-RNA format, compared to the DNA-RNA-DNA format, are that synthesis of the DNA-RNA construct may be facilitated, especially using ligation or enzymatic polymerization approaches to synthesize the HARP probe.

The above descriptions illustrate that alternative designs for the HARP probes used in the present invention may be used, which differ in the size and relative position of the modifiable residues (*e.g*. RNA bases) within the HARP probe, and that differ in the basic format (*e.g*. DNA-RNA-DNA vs DNA-RNA). There are advantages and drawbacks for each particular design. For example, HARP probes designed to include only one or a few bases of RNA are less susceptible to nonspecific RNase cleavage when hybridized to their complementary targets, but may have a higher background signal in the absence of target. Conversely, probes designed to have longer RNA regions extending over most of the target-complementary region may be more susceptible to over-digestion when hybridized to target, but are expected to show a lower background signal (less "noise") in the absence of target. Another important feature of the HARP probe design relates to the position of the Probe Inactivation Region, i.e. the modifiable bases (e.g. RNA bases) relative to the position of the Detection Region, e.g. a binding site for a dual-labeled fluorescent probe ("TAQMAN probe"), which can affect both sensitivity and specificity for target detection. It will be appreciated by those with skill in the art that many alternative formats for design of HARP probe are possible and are within the scope of the present invention.

### G. HARP Probes for Multiplex HARP Assays

Multiplex detection of multiple targets is specifically contemplated. In such cases, a sample potentially contains multiple target nucleic acid sequences. Consequently, the hybridization mixture may comprise a first HARP probe for a first target nucleic acid sequence, a second HARP probe for a second target nucleic acid sequence, and potentially additional HARP probes for additional target nucleic acid sequences. It is contemplated that there may be 1, 2, ....500 or more different HARP probes in a hybridization mixture. The HARP probes may be different by virtue of their containing different Target Hybridization Regions and/or different Amplification Regions and/or different Detection Regions. Multiple HARP probes may be designed to detect, identify or quantify the same target nucleic acid molecule, using probes having different and/or overlapping Target Hybridization Regions which are complementary to distinct segments of the target, for example which hybridize at multiple positions within a target mRNA. Alternatively, in some embodiments, there are multiple HARP probes that are designed to detect different target sequences in distinct nucleic acid molecules.

When the HARP assay is used to detect multiple target in a multiplex format, detection probes (for example such as TAQMAN probes) can be labeled with the same or different labels, for example different fluorescent labels. HARP probes used for multiplex detection will generally have distinct Target Hybridization Regions and may have common or distinct Amplification Regions, or may have one or more common Amplification Regions and one or more common or distinct Detection Regions such as binding sites for dual-labeled fluorogenic probes. It is contemplated that HARP probes used for multiplex detection may have different Amplification Regions, or they may have 1, 2, 3, 4, 5, 6, 7, 8 or more Amplification Regions that are the same. In certain embodiments, the HARP probes have both at least one common Amplification Region and at least one unique Amplification Region. With the unique Amplification Regions, it is contemplated that primers having a sequence that is completely or partially complementary to different targeted nucleic acid sequences can be employed.

### III. Target Nucleic Acids and Target Nucleic Acid Sequences

The target nucleic acid sequence is not limited to any particular sequence, source, or type. In particular embodiments, the target nucleic acid sequence is from an RNA molecule. The RNA molecule can be selected from the group consisting of mRNA, rRNA, tRNA, miRNA, viral RNA, bacterial RNA, noncoding transcribed RNA, and siRNA. The method of the present invention is particularly effective for amplifying and detecting short sequences. Thus, the target sequence can be or be at most 1, 2, ....500 nucleotides in length, or any range derivable therein. Note: the total length of the molecule which contains the actual target of the HARP probe can be significantly longer than the Target Hybridization Region. For example, a HARP probe can be designed to have a Target Hybridization Region of 25 bases to detect an mRNA target of several kilobases. In other embodiments the target may be a DNA molecule. HARP probes may be particularly useful for detecting and quantifying DNA viruses or for distinguishing targets with single nucleotide polymorphisms (SNPs).

In particular embodiments, a "HARP assay," i.e. an assay involving the use of a HARP probe, may be employed to detect miRNA sequences. Since mature, processed miRNAs are typically 19-24 nucleotides in length, if a processed miRNA sequence is targeted, the Target Hybridization Region of the HARP probe will, in most embodiments, be in that range as well. However, if a miRNA *precursor* is targeted, the Target Hybridization Region may be longer than 19 - 24 nucleotides since miRNA precursors are generally between 62 and 110 nucleotides in humans. The present invention provides for a method to distinguish between mature and precursor miRNAs by construction of HARP probes whose Target Hybridization Regions include bases complementary to residues in the precursor miRNA that are not present in the mature miRNA.

HARP probes can be mixed with a total RNA sample, synthetic RNA or DNA target, or with RNA enriched for small molecules including miRNAs, and incubated under conditions to allow target miRNA to hybridize with a miRNA-complementary region in the HARP probe. In this regard, Target Hybridization Regions in HARP probes can consist of miRNA-complementary regions containing a variable number of bases of RNA (the Probe Inactivation Region) flanked by a variable number of bases of DNA within the Target Hybridization Region. Hybridization reaction volume, time, temperature, and salt concentration may vary according to the amount of sample RNA and the number and sequences of probes used. After hybridization has taken place, the reaction is mixed with a solution containing one or more single-strand-specific RNases, to cleave any unhybridized HARP probe(s) at their single stranded RNA region, *i.e*. at the Probe Inactivation Region. HARP probes that are hybridized to the complementary target (*e.g*. miRNA) will be double-stranded across the Probe Inactivation Region (*i.e*. the RNA portion) and will therefore be resistant to RNase cleavage. HARP probes that survive the RNase digestion step will be amplified, for example by PCR™ and/or by linear amplification via *in vitro* transcription with T7 polymerase. Prior to amplification, the HARP probe may be converted to its complementary sequence by reverse transcription with an appropriate reverse transcriptase enzyme such as MMLV-RT. The amplification products can be detected via any method known to those of skill in the art, including, but not limited to use of "Detection Regions" comprising binding sites for dual-labeled fluorescent probes (for example, "TAQMAN probes") or that allow capture of the amplified product by hybridization to a complementary oligonucleotide immobilized on a solid support such as a glass slide microarray or a bead. The Detection Region may consist of the Target Hybridization Region (i.e. the target sequence) or its complement, or of a sequence distinct from the Target Hybridization Region that is included in the HARP probe. For multiplex detection, each HARP probe can have a distinct Detection Region sequence to allow amplification products from distinct HARP probes to be captured onto distinct addressable beads or distinct elements of arrays or microarrays.

Specific methods of the invention also include a method for detecting one or more target nucleic acid sequences using the HARP assay comprising: a) mixing, under hybridization conditions, at least a first nucleic acid molecule comprising the first target nucleic acid sequence with one or more HARP probes to form a hybridization mixture, wherein the HARP probe comprises a Target Hybridization Region and at least one Amplification Region; b) exposing the HARP probe(s) to one or more modifying agents that modifies the Probe Inactivation Region within the Target Hybridization Region of the HARP probe(s) if said probe(s) is/are not hybridized to the target nucleic acid; and, c) then amplifying the intact HARP probe(s) remaining after the modification step, wherein the first target nucleic acid sequence is detected. Sample Preparation

It is contemplated that nucleic acids from of a wide variety of samples can be analyzed using the technology of the invention. While endogenous nucleic acids are contemplated as targets that can be identified, quantitated, or detected with compositions and methods of the invention, recombinant nucleic acids can also be handled and analyzed as described herein. Samples used with methods of the invention are not limited in any way. Samples may be biological samples, in which case, they can be from blood, tissue, organs, semen, saliva, tears, urine, cerebrospinal fluid, synovial fluid, pancreatic fluid, other bodily fluid, hair follicles, skin, or any sample containing or constituting biological cells. Alternatively, the sample may not be a biological sample, but be a chemical mixture, such as a cell-free reaction mixture (which may contain one or more biological enzymes). Moreover, the sample may be fresh, have been frozen or fixed in aldehyde or another fixative and/or embedded in paraffin or other embedding medium.

Prior to detecting or quantitating a targeted nucleic acid using methods of the invention, steps can be taken to isolate or purify the targeted nucleic acid. For example, a cell lysate can be prepared when the targeted nucleic acid is from a sample containing cells. A number of methods are well known to those of skill in the art and reagents are commercially available such as Ambion's mirVana™ miRNA Isolation Kit, Poly(A)Pure™ and MicroPoly(A)Pure™ Kits, Poly(A)Purist™ mRNA Purification Kit, ToTALLY RNA™ Kit, RiboPure kit, RiboPure-Blood kit, RNAqueous kit, RNAqueous-Micro kit, RNAqueous4PCR™ kit, RNAqueous-Midi kit, Optimum kit for FFPE samples, and RiboPure-bacteria kit. Furthermore, samples may have been prepared for laser capture microdissection, such as is described in U.S. Patent Application Ser. No. 10/462,091, Methods and Compositions for Preparing Tissue Samples for RNA Extraction. The invention can also be used with fixed or degraded tissue, whose preparation is described in U.S. Serial No. 10/899,386, entitled, Methods and Compositions for Preparing RNA from Fixed Samples.

Nucleic acids may be isolated using techniques well known to those of skill in the art, and methods for isolating small nucleic acid molecules and/or isolating RNA or DNA molecules can be employed. However, a particular benefit of the method of the present invention is that due to the increased sensitivity afforded by the amplification step, enrichment of small nucleic acid molecules is generally not necessary. This results in significant savings in cost and labor associated with sample preparation. If RNA from cells is to be used or evaluated, methods generally involve lysing the cells with a chaotropic (e.g., guanidinium isothiocyanate) and/or detergent (e.g., N-lauroyl sarcosine) prior to implementing processes for isolating particular populations of RNA.

### Small RNA Molecules

The present invention has particular use in regard to the analysis of a wide variety of small RNA molecules, which are not amenable to analysis via many prior forms of assays. Such small RNA molecules include, but are not limited to, miRNAs, siRNAs, and degraded RNAs such as RNAs from fixed tissues wherein the fixation has resulted in RNA degradation. Of course, the invention is useful in the context of many lengths of RNA and is in no way limited only to embodiments involving shorter RNAs

In the context of the invention, a "small RNA" may be generally defined as RNA molecules of less than about 100 bases. Small RNAs may be single-stranded or double-stranded miRNA molecules (miRNAs) are particularly contemplated as a nucleic acid molecule that can be targeted using methods and compositions of the invention. Traditionally, differential gene expression studies have been concerned with the analysis of messenger RNA (mRNA) patterns but more recently expression studies have been carried out to detect and quantify miRNAs. miRNAs are a recently-discovered class of short single-stranded RNA molecules of - 22 bases in length, which are partially complementary to sequences in the 3' untranslated region of mRNAs. miRNAs have been identified in both plants and animals, including lower eukaryotes and mammals (humans and mice). Recent reports suggest that miRNAs are involved in developmental regulation of gene expression and that their levels are differentially regulated in pathological conditions including leukemia and possibly cancer. See U.S. Provisional Pat. App. U. S. Provisional Patent Application No: 60/575,743, entitled "Methods and Compositions Involving MicroRNA". miRNAs have also been identified in differentiating neurons and are proposed to play a role in neuronal plasticity involved in learning and memory.

The term "miRNA" is used according to its ordinary and plain meaning and refers to a miRNA molecule found in eukaryotes that is involved in RNA-based gene regulation. *See, e.g.,* Carrington *et al.,* 2003. Individual miRNAs have been identified and sequenced in different organisms, and they have been given names. Names of miRNAs and their sequences are provided herein. Additionally, other miRNAs are known to those of skill in the art and can be readily implemented in embodiments of ' the invention. The methods and compositions should not be limited to miRNAs identified in the application, as they are provided as examples, not as limitations of the invention.

miRNA molecules are generally 21 to 22 nucleotides in length, though lengths of 19 and up to 23 nucleotides have been reported. The term "miRNA," unless otherwise indicated, refers to the processed RNA, after it has been cleaved from its precursor. The miRNAs are each processed from a longer precursor RNA molecule ("precursor miRNA"). Precursor miRNAs can be transcribed from non-protein-encoding genes. The precursor miRNAs have two regions of complementarity that enable them to form a stem-loop- or fold-back-like structure, which is cleaved- by an enzyme called Dicer in animals. Dicer is ribonuclease III-like nuclease. The processed miRNA is typically a portion of the stem.

Techniques for manipulating miRNA can be found in U.S. Application Serial No. 10/667,126, entitled "Methods and Compositions for Isolating Small RNA Molecules" and U. S. Provisional Patent Application No: 60/575,743 entitled "Methods and Compositions Involving MicroRNA".

The general scheme of the invention when an miRNA sequence is targeted involves the following: HARP probe(s) is/are mixed with total RNA sample (or with RNA enriched for small size including miRNAs) and incubated under conditions to allow target RNA to hybridize with the Target Hybridization Region in the HARP probe. This is generally about 20 bases in length. Optimal hybridization conditions may require co-precipitation of the probe(s) with the sample RNA, and may require that hybridization volume is kept to a minimum, e.g., approximately 3 - 10 µL. Hybridization time and temperature and salt concentration may vary according to the amount of sample RNA and the number and sequences of probes used. After hybridization has taken place, the reaction will be diluted with a solution containing one or more single-strand-specific RNases, which will cleave the remaining unhybridized HARP probe(s) at their Probe Inactivation Regions. The RNase digestion buffer will contain a concentration of monovalent cation or other salts or other components sufficient to maintain the target RNA/HARP probe as a stable duplex. HARP probes that are hybridized to the complementary target RNA(s) will be double-stranded across the Target Hybridization Region, including the Probe Inactivation Region (*i.e*. the RNA portion of the Target Hybridization Region), and will therefore be resistant to RNase cleavage. HARP probes that survive the RNase digestion step will be amplified by PCR™, by linear amplification with T7 polymerase or other amplification methods. The amplification products could be detected by their Detection Regions, for example regions that hybridize to dual-labeled fluorescent probes ("TAQMAN probes"), or by capture via their Target Hybridization Regions onto an immobilized solid support such as a glass slide or nylon filter oligonucleotide microarray, on onto an array of addressable microbeads such as Luminex beads. The amplification products could be captured onto Luminex beads using bead-coupled oligos complementary to the unique miRNA sequences themselves, or the amplification products could be captured onto beads using zip code or barcode strategy (U.S. Patent Application No. 20030170623), provided the HARP probes were designed to include Detection Regions comprising the appropriate additional sequences (*i.e*., zip code-complementary sequences).

RNA molecules from formaldehyde fixed paraffin embedded tissue (FFPE) are particularly contemplated as a nucleic acid molecule that can be targeted using methods and compositions of the invention. FFPE RNA is frequently highly degraded and presents a small target size. HARP probes are one of the few methods that are ideally suited to small targets.

### V. Hybridization

Hybridization between the Target Hybridization Region of a probe and a targeted nucleic acid can occur under the appropriate conditions, which can be adjusted, as desired, to provide for an optimum level of specificity in view of the particular assay being implemented. Suitable hybridization conditions are well known to those of skill in the art and reviewed in Sambrook *et al.,* 1989 and WO 95/21944.

Moreover, hybridization can be employed during the amplification aspect of the methods. PCR™ involves multiple hybridization steps involving at least one set of primers complementary to the probe sequence. The conditions for achieving amplification are well known to those of skill in the art.

As used herein, "hybridization", "hybridizes" or "capable of hybridizing" is understood to mean the forming of a double or triple stranded molecule or a molecule with partial double or triple stranded nature. The term "anneal" as used herein is synonymous with "hybridize."

For nucleic acids, one of skill in the art can readily determine experimentally the features (such as length, base composition, and degree of complementarity) that will enable a nucleic acid (Target Hybridization Region of probe) to hybridize to another nucleic acid (targeted nucleic acid sequence) under conditions of selected stringency, while minimizing non-specific hybridization to other substances or molecules.

### VI. Modifying Agents

Methods of the invention involve carrying out the HARP assay by exposing the HARP probe, after being incubated with a target nucleic acid sequence under hybridization conditions, to a modifying agent. The term "modifying agent" refers to a substance that can introduce a chemical change into or onto a susceptible ("modifiable") residue or residues of the probe (or between two residues) when the susceptible residue(s) is/are not specifically hybridized to a target nucleic acid. In certain embodiments, the modifying agents can modify unhybridized residue(s) by enzymatically or chemically cleaving the HARP probe at that position(s). Alternatively, the modifying agent may chemically alter the unhybridized residue such that subsequent amplification is blocked. The invention is not limited to certain modifying agents or modifications, so long as the probe is altered to distinguish hybridized sequences from unhybridized sequences in the Target Hybridization Region.

In other embodiments, the modification of the unhybridized HARP probe(s) need not involve cleavage of the probe(s), so long as the unhybridized probe is rendered completely or substantially incompetent for amplification in the selected amplification system. For example, if the unhybridized HARP probe comprises in its Target Hybridization Region a native or modified nucleotide that can be modified by attachment to a chemical moiety that would prevent a polymerase required for amplification from utilizing the modified unhybridized probe as template for amplification, then cleavage would not be required. Of course, those of skill in the art will, in view of the teachings herein, be able to devise such non-cleavage based systems of inactivation.

### A. Modifying Agents Comprising Nucleases

The present invention involves modifying agents that can distinguish between hybridized residues and unhybridized residues. Some nucleases have as substrates only nucleotides that are not hydrogen bonded to a complementary nucleotide, that is, they are specifically able to cleave single-stranded but not double-stranded nucleic acids. Nucleases may have specificity for deoxyribonucleotides versus ribonucleotides. Both types of nucleases can be used in methods of the invention so long as they distinguish hybridized from unhybridized residues. In the context of this aspect of the invention, the term "nuclease" encompasses any polypeptide that functions to cleave nucleic acid, whether or not that polypeptide is generally termed a "nuclease," "ribonuclease," and/or "deoxyribonuclease.

### 1. Ribonucleases

Ribonucleases compatible with methods of the invention are generally single-strand specific and include, but are not limited to, RNase A, RNase T1, and RNase 1. These RNases are readily available and their activities well known. Ribonuclease H is not an appropriate modifying agent because hybridized residues are substrates for this enzyme.

### 2. Deoxyribonuclease

Nucleases for which DNA is a substrate can also be used. Appropriate DNases will generally be single-strand-specific. S1 nuclease is an example that is also readily available.

### B. Chemical Agents

It is contemplated that the invention can be carried out using chemical cleavage rather than enzymatic cleavage to eliminate unhybridized probes after the hybridization step. In one format, a standard HARP probe would be used that contained one or more RNA bases in the Probe Inactivation Region, but after the hybridization step these bases would be cleaved by a non-enzymatic treatment that specifically (or at least preferentially) cleaved the phosphodiester bonds in single-stranded RNA. A chemical treatment that could potentially be used to cleave single-stranded RNA or DNA is carbodiimide, which is reported to react with unpaired bases in uracil and guanine ribonucleotides (Smooker and Cotton, 1993). Other chemicals potentially useful for modifying single-stranded DNA and/or RNA are osmium tetroxide, hydroxylamine, hydrazine, diethylpyrocarbonate, methylene blue, formic acid, potassium permanganate, and sodium hydroxide; after treatment with these agents, the nucleic acid can be cleaved at the modified positions by treatment with piperidine (Smooker and Cotton, 1993.)

### C. Inactivation of Modifying Agents

Some embodiments of the invention include inactivating the modifying agent prior to amplifying the HARP probe. A protease enzyme such as Proteinase K and/or heat can be used to inactivate the modifying agent, for example to inactivate RNase. Alternatively RNase inhibitors could be used to inactivate the RNase. The advantage of inactivating the RNase after the modification step of the assay (*i.e*. after the cleavage step) is to prevent cleavage of the HARP probe that survived the RNase treatment due to being hybridized to its target nucleic acid, when the HARP probe and target are subsequently denatured during synthesis of the complementary strand by reverse transcriptase and/or during the first cycle of the PCR™ amplification reaction.

### VII. Amplification of Nucleic Acids

Amplification can be achieved by many methods familiar to those skilled in the art, including PCR™, transcription with T7 RNA polymerase, NASBA, Strand Displacement Amplification (SDA), etc. While PCR™ is a currently preferred manner of amplification, those skilled in the art will appreciate that alternative methods besides PCR™ exist for amplifying the HARP probe. For example, appropriately designed HARP probes can be amplified by *in vitro* transcription using a phage RNA polymerase such as T7, T3, or Sp6 RNA polymerase to synthesize multiple copies of the complement of the HARP probe initiated from a corresponding phage promoter which comprises an Amplification Region of the HARP probe. Other amplification technologies such as Strand Displacement Amplification (SDA), Transcription Mediated Amplification (TMA), and nucleic acid sequence based amplification (NASBA) can also be used. Virtually any amplification technology in which the amplification step can be blocked by cleavage in the Probe Inactivation Region of the HARP probe is compatible with the present invention. In the context of the specific embodiments of the invention that discuss the use of PCR™ in this specification, those of skill will be able to adapt those embodiments to use any other suitable amplification procedure and will understand that this specification describes the use of any suitable amplification procedure in the practice of those specific embodiments.

The term "primer," as used herein, is meant to encompass any nucleic acid that is capable of priming the synthesis of a nascent nucleic acid in a template-dependent process. Typically, primers are oligonucleotides ranging from twenty to thirty base pairs in length, but longer sequences can be employed. For amplification using PCR™, pairs of primers are designed to selectively hybridize to nucleic acids corresponding to probe Amplification Regions under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids contain one or more mismatches with the primer sequences. Once hybridized, the template-primer complex is contacted with one or more enzymes that facilitate template-dependent nucleic acid synthesis. Multiple rounds of template denaturation, primer annealing, and enzymatic polymerization, also referred to as "cycles," are conducted until a sufficient amount of amplification product is produced.

Oligonucleotide primers that may be used to prime synthesis of the complementary sequence of the HARP probe during the initial stages of amplification, may be designed such that their 3' ends extend past the RNA region of the HARP probes. Such a design is beneficial for avoiding the need to use a reverse transcriptase step, which may be less efficient than DNA polymerase for quantitative synthesis of the complements of nucleic acids and which have other limitations in terms of cost and labor. Using primers that bypass the RNA region of HARP probes also avoids the need for a DNA polymerase (for example, Taq polymerase) to use RNA bases as template; synthesis of RNA-templated product by DNA polymerases may be less efficient than synthesis of the corresponding DNA-templated product.

The amplification product may be detected or quantified. In certain applications, the detection may be performed by visual means. Alternatively, the detection may involve indirect identification of the product via chemiluminescence, radioactive scintigraphy of incorporated radiolabel or fluorescent label or even via a system using electrical and/or thermal impulse signals (Bellus, 1994).

A number of template dependent processes are available to amplify the oligonucleotide sequences present in a given template sample. One of the best known amplification methods is PCR™, as is described in detail in U.S. Patents 4,682,195, 4,683,202 and 4,800,159, and in Innis *et al.,* 1988.

A reverse transcriptase PCR™ (RT-PCR) amplification procedure may be performed to amplify targets containing RNA bases. Methods of reverse transcribing RNA into cDNA are well known (see Sambrook *et al.,* 1989). Alternative methods for reverse transcription utilize thermostable DNA polymerases. These methods are described in WO 90/07641. PCR™ methodologies are well known in the art. Representative methods of RT-PCR are described in U.S. Patent 5,882,864.

Alternative methods for amplification of target nucleic acid sequences that may be used in the practice of the present invention are disclosed in U.S. Patents 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, GB Application No. 2 202 328, and in PCT Application No. PCT/US89/01025.

Qbeta Replicase, described in PCT Application No. PCT/US87/00880, may also be used as an amplification method in the present invention. In this method, a replicative sequence of RNA that has a region complementary to that of a target is added to a sample in the presence of an RNA polymerase. The polymerase will copy the replicative sequence that may then be detected.

An isothermal amplification method, in which restriction endonucleases and ligases are used to achieve the amplification of target molecules that contain nucleotide 5'-[alpha-thio]-triphosphates in one strand of a restriction site, may also be useful in the amplification of nucleic acids in the present invention (Walker *et al.,* 1992). Strand Displacement Amplification (SDA), disclosed in U.S. Patent 5,916,779, is a method of carrying out isothermal amplification of nucleic acids that involves multiple rounds of strand displacement and synthesis, *i.e*., nick translation.

Other nucleic acid amplification procedures include transcription-based amplification systems (TAS), including nucleic acid sequence based amplification (NASBA) and 3SR (Kwoh *et al*., 1989; PCT Application WO 88/10315, ). European Application No. 329 822 discloses a nucleic acid amplification process involving cyclically synthesizing single-stranded RNA ("ssRNA"), ssDNA, and double-stranded DNA (dsDNA), which may be used in accordance with the present invention.

PCT Application WO 89/06700 disclose a nucleic acid sequence amplification scheme based on the hybridization of a promoter region/primer sequence to a target single-stranded DNA ("ssDNA") followed by transcription of many RNA copies of the sequence. This scheme is not cyclic, *i.e.,* new templates are not produced from the resultant RNA transcripts.

### I.Nucleic Acid Detection and Analysis/Discrimination of Sequence Differences

Methods of the invention will generally include detecting the products of the amplified HARP probes and/or quantitating the products of the amplified HARP probes, both of which are familiar to a person of ordinary skill in the art.

The invention can be used to detect differences between nucleic acid sequences. Specifically contemplated applications include identifying and/or quantifying differences between the same gene or transcript but from different samples, such as mutations in the nucleic acid sequence or polymorphisms in the nucleic acid sequence.

Assays can have diagnostic or prognostic value. For example, a nucleic acid sequence from a sample obtained from a subject believed to be susceptible to or having a particular disease or condition can be evaluated with respect to a sequence from a sample obtained from a subject believed or known to be not susceptible or resistant to that disease or condition. A sample that is not normal is one exhibiting phenotypic trait(s) of a disease or condition or one believed to be not normal with respect to that disease or condition.

### A. Nucleic Acid Labeling and Detection

The use of labels for nucleic acids, such as fluorescent ones, are specifically contemplated for use with the present invention. Labels on nucleic acids may be colorimetric (includes visible and UV spectrum, including fluorescent), luminescent, enzymatic, or positron emitting (including radioactive). The label may be detected directly or indirectly. Radioactive labels include ¹²⁵I, ³²P, ³³P, and ³⁵S. Examples of enzymatic labels include alkaline phosphatase, luciferase, horseradish peroxidase, and β-galactosidase. Labels can also be proteins with luminescent properties, *e.g*., green fluorescent protein and phycoerythrin. Labels may comprise haptens such as digoxigenin or moieties such as biotin.

A number of techniques for visualizing or detecting labeled nucleic acids are readily available. The reference by Stanley T. Crooke, 2000 has a discussion of such techniques (Chapter 6). Such techniques include, microscopy, arrays, Fluorometry, Light cyclers or other real time PCR™ machines, FACS analysis, scintillation counters, Phosphoimagers, Geiger counters, MRI, CAT, antibody-based detection methods (Westerns, immunofluorescence, immunohistochemistry), histochemical techniques, HPLC (Griffey *et al.,* 1997, spectroscopy, capillary gel electrophoresis (Cummins *et al.,* 1996), spectroscopy; mass spectroscopy; radiological techniques; and mass balance techniques. Nucleic acids can also be labeled by interaction with intercalating dyes such as ethidium bromide and detected by fluorescence under ultraviolet light after electrophoretic separation, for example on agarose or acrylamide gels.

When two or more differentially colored labels are employed, fluorescent resonance energy transfer (FRET) techniques may be employed to characterize the dsRNA. Furthermore, a person of ordinary skill in the art is well aware of ways of visualizing, identifying, and characterizing labeled nucleic acids, and accordingly, such protocols may be used as part of the invention. Examples of tools that may be used also include fluorescent microscopy, a BioAnalyzer, a plate reader, Storm (Molecular Dynamics), Array Scanner, FACS (fluorescent activated cell sorter), a Luminex microbead scanner, or any instrument that has the ability to excite and detect a fluorescent molecule.

### B. TAQMAN qRT-PCR Assays

In one preferred embodiment of the present invention, the amplification products of the HARP probe are detected in a quantitative manner using TAQMAN probes in a qRT-PCR assay. TAQMAN probes are described in U.S. Patent No. 5,723,591. The TAQMAN probes take advantage of the 5'-->3' exonuclease activity of *Taq* DNA polymerase. The TAQMAN probe (generally 20-30 bp), which has been engineered to prevent extension at the 3' end, consists of a site-specific sequence labeled with a fluorescent reporter dye and a fluorescent quencher dye. During PCR™, the TAQMAN probe hybridizes to its complementary target sequence. When amplification occurs the TAQMAN probe is degraded due to the 5'-->3' exonuclease activity of *Taq* DNA polymerase, thereby separating the quencher from the reporter during extension. The reporter can then be measured. The amount of light emitted increases exponentially, and the final amount can be measured by spectrophotometry after termination of the reaction. Modifications of TAQMAN probes such as minor groove binding moieties may provide advantages for use in the present invention and have in fact been used for improving sensitivity and specificity of detection of the HARP probes in the present invention.

Fluorescent probes other than TAQMAN probes can also be used for qPCR; examples of such probes are molecular beacons and molecular scorpions. Another way in which qPCR can be carried out is by monitoring the signal from an intercalating dye such as Sybr Green (Molecular Probes, Inc.) that selectively (or at least preferentially) binds to and generates a fluorescent signal in the presence of double-stianded DNA. As amplified product accumulates, additional dye is bound and additional signal generated. A Ct value that reflects the initial amount of target nucleic acid is assigned as described above.

The Cycle Threshold (Ct) is the cycle number in the PCR™ where a sufficient amount of amplified product has been produced to generate a fluorescent signal that is high enough to cross a threshold determined by the end-user. A cycle threshold value (Ct value) is assigned to each reaction in the qPCR™ assay. The Ct value is set in the exponential amplification phase of the PCR™, *i.e.* in that portion of the reaction in which the amount of amplified product approximately doubles at each cycle. The greater the initial amount of target, the fewer cycles are needed to generate sufficient signal to cross the threshold. Therefore the lower the Ct value, the greater the amount of target (for example HARP probe) was initially present. For amplification reactions in which the amount of amplified product doubles at each cycle (*i.e*. those having the theoretical maximum efficiency), a difference of 3.3 in Ct value between two reactions corresponds to a 10-fold difference in initial concentration of target nucleic acid. qPCRs are generally designed such that the amplified products are quite short, approximately 100 bp, in order to maximize the efficiency of the reaction and approach this theoretical efficiency.

As set forth above, the invention provides some particular benefits over prior methods in the context of small RNA targets, such as miRNA, siRNA, and degraded RNA. However, the use of the invention is not limited to short RNAs. Below is described a series of embodiments using HARP probes in the context of qPCR and qRT-PCR assays for miRNA. Of course, those of skill, in view of this specification, will be able to adapt the steps below for use with any other form of RNA target or DNA target, without undue experimentation.

In order to detect miRNA with a HARP assay, HARP probes can be mixed with total RNA sample (or with synthetic RNA or DNA targets or with RNA enriched for small molecules including miRNAs) and incubated under conditions to allow target miRNA to hybridize with a micro-RNA-complementary Target Hybridization Region in the HARP probe, for example, a 22-base micro-RNA-complementary Target Hybridization Region in the HARP probe.

In this regard, Target Hybridization Regions in HARP probes can consist of miRNA-complementary regions containing a variable number of bases of RNA (the Probe Inactivation Region) flanked by a variable number of bases of DNA within the Target Hybridization Region. Optimal hybridization conditions may require co-precipitation of the probe(s) with the sample RNA, for example by adding 2 volumes of ethanol to the sample and centrifuging it to pellet the RNA and then removing the supernatant fluid, so that hybridization volume is kept to a minimum. Hybridization time and temperature and salt concentration may vary according to the amount of sample RNA and the number and sequences of probes used. After hybridization has taken place, the reaction is mixed with a solution containing one or more single-strand-specific RNases, which will modify, that is cleave, the remaining unhybridized HARP probe(s) at their Probe Inactivation Region, i.e. the single stranded RNA region. HARP probes that are hybridized to the complementary target (*e.g*. miRNA) will be double-stranded across the Probe Inactivation Region (i.e. the RNA portion) and will therefore be resistant to RNase cleavage. HARP probes that survive the RNase digestion step will be amplified by PCR™ and/or by linear amplification with T7 polymerase.

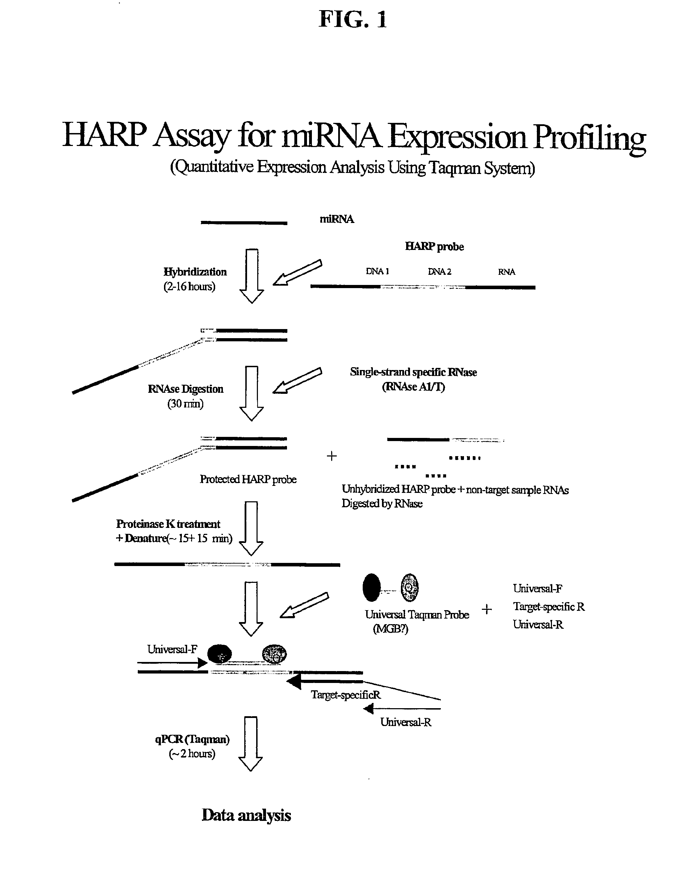

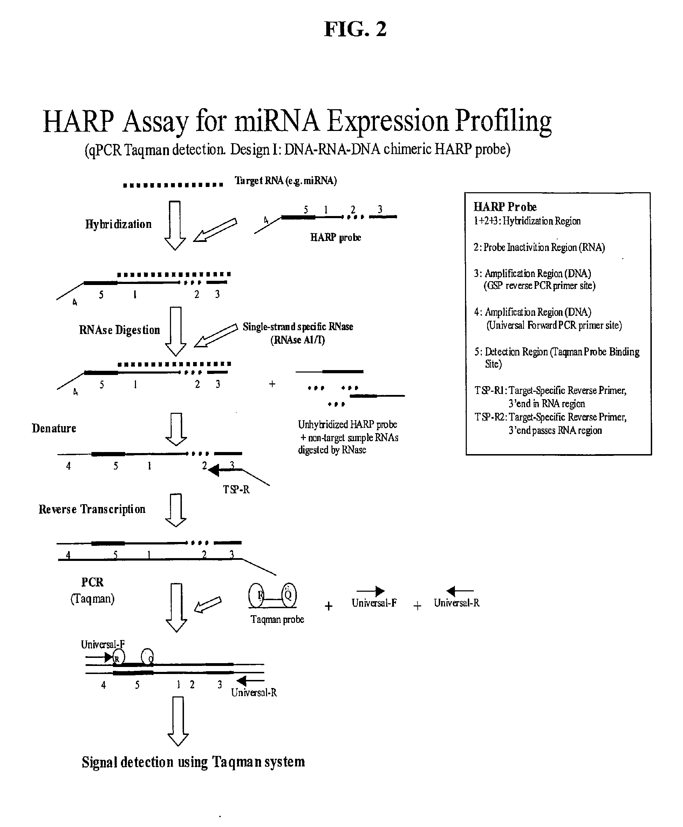

FIG. 1 and FIG. 2 show exemplary quantitative expression analysis embodiments of the invention.

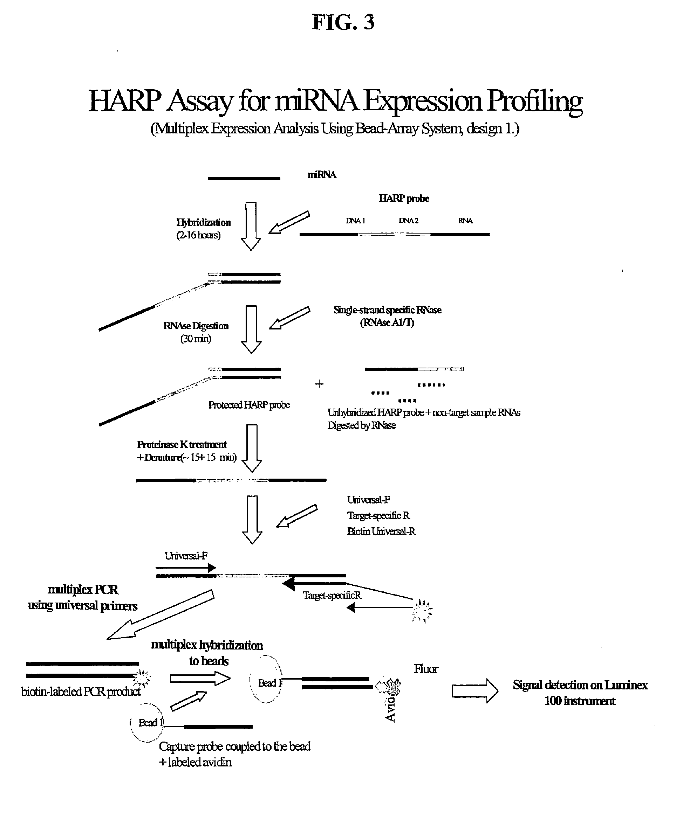

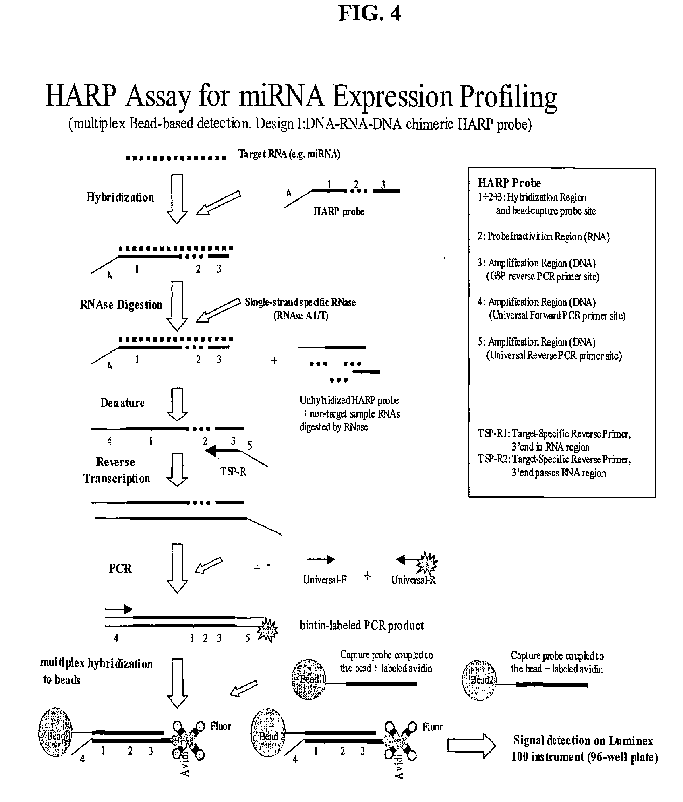

FIG. 3 and FIG. 4 show exemplary multiplex detection embodiments of the invention where, each HARP probe can have a distinct Detection Region sequence to allow amplification products from distinct HARP probes to be captured onto distinct addressable beads or distinct elements of arrays or microarrays.

Prior to amplification, the HARP probe may be converted to its complementary sequence by reverse transcription with an appropriate reverse transcriptase enzyme. This is shown in FIG. 2 and FIG. 4.

The amplification products can be detected by "Detection Regions" which may consist of the Target Hybridization Region (*i.e*. the target sequence) or its complement, or of a sequence distinct from the Target Hybridization Region that is included in the HARP probe. As shown in FIG. 1 and FIG. 2, this detection may be accomplished by binding sites for dual-labeled fluorescent probes (for example, the TAQMAN probes described above) that hybridize to the Detection Regions. Alternatively, as shown in FIG. 3 and FIG. 4, the Detection Regions may allow capture of the amplified product by hybridization to a complementary oligonucleotide immobilized on a solid support such as a glass slide microarray or an addressable Luminex bead.

Of course, those of skill will recognize that a variety of alternative embodiments of the invention may be realized in an number of manners consistent with the above, including an number of alternative embodiments for assaying nucleic acids other than miRNAs.

### C. Assays for SNPs and Mutations

One method of screening for SNPs and/or point mutations is based on RNase cleavage of base pair mismatches in RNA/DNA or RNA/RNA heteroduplexes. As used herein, the term "mismatch" is defined as a region of one or more unpaired or mispaired nucleotides in a double-stranded RNA/RNA, RNA/DNA or DNA/DNA molecule. This definition thus includes mismatches due to insertion/deletion mutations, as well as single or multiple base point mutations.

U.S. Patent 4,946,773 describes an RNase A mismatch cleavage assay that involves annealing single-stranded DNA or RNA test samples to an RNA probe, and subsequent treatment of the nucleic acid duplexes with RNase A. For the detection of mismatches, the single-stranded products of the RNase A treatment, electrophoretically separated according to size, are compared to similarly treated control duplexes. Samples containing smaller fragments (cleavage products) not seen in the control duplex are scored as positive.

Other investigators have described the use of RNase I in mismatch assays. The use of RNase I for mismatch detection is described in literature from Promega Biotech. Promega previously marketed a kit containing RNase I that was reported to efficiently cleave basepair mismatches. Others have described using a DNase isolated from celery for cleavage of all types of basepair mismatches.

Alternative methods for detection of deletion, insertion or substitution mutations that may be used in the practice of the present invention are disclosed in U.S. Patents 5,849,483, 5,851,770, 5,866,337, 5,925,525 and 5,928,870 All of the mutation detection methods described above may be used with HARP probes.

### D. Solid Supports and Arrays

Methods of the invention can be performed using solid-supports of various types such as microarrays, or using liquid microbead arrays (such as those sold by Luminex), to detect the amplified products of multiple distinct HARP probes. Elements of such arrays may comprise sequences partially or completely *complementary* to Target Hybridization Regions of distinct HARP probes, or sequences partially or completely *identical* to Target Hybridization Regions of distinct HARP probes. Elements of the arrays may also or alternatively comprise sequences complementary or identical to distinct Detection Regions of distinct HARP probes. Detection Regions used for microarray-based or liquid microbead array-based detection of amplified products of HARP probes, are sequences that are unique to distinct HARP probes and that can be used to associate the amplified products of distinct HARP probes with distinct addressable elements on solid-support arrays or with distinct addressable microbeads. Arrays may comprise ordered arrays of oligonucleotides (also known as "elements") attached to a two-dimensional solid support such as glass, plastic, nylon, or other solid supports, including arrays of oligonucleotides attached to spherical supports such as microbeads.

### 1. Microarrays

The present invention can be used with arrays, including ordered macroarrays or microarrays of nucleic acid molecules (probes) that are complementary or identical to a plurality of targeted molecules that are positioned on a support material in a spatially separated organization. Representative methods and apparatus for preparing a microarray have been described extensively in the literature. A person of ordinary skill in the art can readily analyze data generated using an array. Such protocols are disclosed above, and include information found in WO 9743450; WO 03023058; WO 03022421; WO 03029485; WO 03067217; WO 03066906; WO 03076928; WO 03093810; WO 03100448A1.

### 2. Detection of Amplification Products via Liquid Microbead Array

Liquid microbead arrays can also be used for detection of the amplified HARP probes of the present invention, and are especially suited for multiplex detection of multiple targets. Liquid microbead arrays, such as those commercialized by Luminex Inc., consist of populations of beads whose members are distinguishable from each other by incorporation of fluorescent dyes that can be differentially detected. In the case of the microbead arrays developed by Luminex, a set of up to one hundred distinct types of beads are created by incorporating two different dyes whose ratio differs for each type of bead, providing a "spectral address" that permits each bead to be identified and distinguished from the other beads. The individual types of beads are coupled to "capture reagents" such as antibodies or oligonucleotides, that permit them to specifically associate with various analytes, and in this way, a mixture of beads can be used to quantitatively detect a mixture of analytes. For the detection step, a solution containing the mixture of beads that has been allowed to react with a test solution potentially containing the analytes of interest (*e.g.* the "liquid bead array") is passed across a photodetector in such a way that the spectral signal and the signal associated with any analyte captured on the bead can be determined for each single bead. Signals associated with analytes can be introduced by labeling the analytes, for example in the case of nucleic acid analysis, the target analytes can be labeled by introducing a detectable moiety into a primer used to amplify the target nucleic acid by PCR™, or by incorporating a dye-modified nucleotide into an enzymatically synthesized nucleic acid. In the case of the present invention, the liquid microbead array can be used to detect the amplified products of HARP probes. The capture oligonucleotides coupled to the beads can be directed against target-specific sequences or against non-target-specific sequences in the amplified targeting probe.

### IX. Kits

Any of the compositions described herein may be comprised in a kit. Various embodiments of the invention may be practiced by way of a kit. Reagents and materials for preparing a HARP probe can be included in a kit. A solid support to attach to the HARP probes can be included in a kit. Kits may also include one or more reagents for isolating or purifying total RNA or miRNA from a sample.

In non-limiting examples, kits would contain one or more HARP probes, hybridization solution, RNase digestion buffer, appropriate RNase(s), Proteinase K solution, and potentially HARP probes for constitutively expressed targets such as 5S ribosomal RNA, to be used for normalization of sample amount. Optional additional reagents are natural or synthetic target nucleic acids such as 22-base miRNAs or 22-base DNA oligos having the corresponding sequence (for use as positive controls), nuclease-free water, concentrated salt solution, and oligonucleotide primers and other reagents for PCR™, RT-PCR, qPCR, and qRT-PCR, or for *in vitro* transcription with RNA polymerase. The kits would also include an instruction manual. The kit may further include reagents for creating or synthesizing HARP probes. The kits may comprise, in suitable container, an enzyme and other reagents for labeling the amplified products of the protected HARP probe by incorporating labeled nucleotide or unlabeled nucleotides that are subsequently labeled. It may also include one or more buffers, such as reaction buffer, labeling buffer, washing buffer, or a hybridization buffer, compounds for preparing the HARP probes, and components for isolating targeted nucleic acids, such as miRNA. Other kits of the invention may include components for making a nucleic acid array comprising the targeted nucleic acids, and thus, may include, for example, a solid support.

Specific exemplary kits may comprise one or more of the following kit components, with component volumes dependent upon on how many reactions are included per kit: Hybridization Solution from the Ambion RPA III Kit, [Cat # 1414]; 200 mM sodium chloride; stock solutions to make working RNase digestion mix (composition of RNase digestion mix: 10 ul of 280 mM KCl, 0 - 0.33 ul of RNase 1 (100 units/ul), 0 - 0.5 ul RNase A/T1 cocktail (Ambion cat #2286); 0 - 3 ul of RNase T1 (Ambion cat # 2280), nuclease free dH₂O; 4. 1 - 2 ml of Proteinase K (20 mg/ml); the HARP probes (chimeric oligonucleotides) designed for detection of distinct target nucleic acids; and primers for amplification of the HARP probes. Kits may also contain TAQMAN probes, Luminex beads, and biotinylated PCR™ primers.

Kits components may be individually packaged or placed in a container, such as a tube, bottle, vial, syringe, or other suitable container means.

Individual components may also be provided in a kit in concentrated amounts; in some embodiments, a component is provided individually in the same concentration as it would be in a solution with other components. Solutions in the kit may have a concentration that is 0.05, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 3.0, 3.5, 4.0, 4.5, 5.0, 5.5, 6.0, 6.5, 7.0, 7.5, 8.0, 8.5, 9.0, 9.5, 10.0 mM or M (or any range derivable therein). Moreover, a solution may be provided in the following concentration 2x, 3x, 4x, 5x, 10x, 20x, 30x, 40x, 50x, 60x, 70x, 80x, 90x, 100x, 110x, 120x, 130x, 140x, 150x, 160x, 170x, 180x, 190x, 200x, 210x, 220x, 230x, 240x, 250x, 260x, 270x, 280x, 290x, 300x, 310x, 320x, 330x, 340x, 350x, 360x, 370x, 380x, 390x, 400x, 410x, 420x, 430x, 440x, 450x, 460x, 470x, 480x, 490x, 500x, 600x, 700x, 800x, 900x, 1000x, 1100x, 1200x, 1300x, 1400x, 1500x, 1600x, 1700x, 1800x, 1900x, 2000x, 3000x, 4000x, 5000x, 6000x, 7000x, 8000x, 9000x, 10,000x or more, or any range derivable therein and it would be diluted to achieve a 1x concentration in the reaction or mixture.

Other solutions that may be included in a kit are those solutions involved in isolating and/or enriching miRNA from a mixed sample. A lysis solution may comprise a chaotropic salt, a detergent, a salt, and/or a reducing agent. In certain embodiments, a lysis solution contains one or more of the following: about 1, 2, 3, 4, 5, 6, 7, or 8 M guanidinium thiocyanate (or any range derivable therein); about 0.1, 0.2, 0.3, 0.4, 0.5, 0.6, 0.7, 0.8, 0.9, 1.0 % or more N lauryl sarcosine (or any range derivable therein); about 5, 10, 15, 20, 25, 30, 35, 40, 45, 50 mM or more NaCitrate; and/or, about 0.0.5, 0.1, 0.15, 0.2 M or more 2-mercaptoethanol (or any range derivable therein). Wash solutions that may be contained in kits include wash solutions having a chaotropic salt and ethanol and wash solutions having a salt and buffer. In specific embodiments wash solutions include: about 1.0, 1.1, 1.2, 1.3, 1.4, 1.5, 1.6, 1.7, 1.8, 1.9, 2.0, 2.1, 2.2, 2.3, 2.4, 2.5 M or more guanidinium thiocyanate (or any range derivable therein) and/or 10, 15, 20, 25, 30, 35, 40, 45, 50, 55, 60% or more ethanol (or any range derivable therein). Other wash solutions can include: about 50, 60, 70, 80, 90, 100, 110, 120, 130, 140, 150 mM NaCl (or any range derivable therein); 3.0, 3.5, 4.0, 4.5, 5.0, 5.5, 6.0, 6.5, 7.0 mM or more EDTA (or any range derivable therein); about 5, 10, 15, 20, 25, 30, 35, 40 mM or more Tris (or any range derivable therein).

It is also contemplated that reagents for purifying miRNA using gel or tube electrophoresis can be included in kits of the invention. A variety of microfilters can be used for recovering, purifying, and concentrating the miRNA obtained using gel or tube electrophoresis, and specifically contemplated in some embodiments of the invention is a glass fiber or silica filter column. Solutions that can be used with such microfilters include a binding buffer and/or wash buffer.

Kits for using miRNA arrays of the invention for therapeutic, prognostic, or diagnostic applications are included as part of the invention. Such kits can include a miRNA array, as well as information regarding a standard or normalized miRNA profile for the miRNAs on the array.

Control RNA or DNA is included in some kit embodiments. Control RNA can be natural or synthetic miRNA, which can be used as a positive control. Control HARP probes can be included for detecting endogenous target RNAs such as ribosomal RNA or endogenous short RNAs such as RNAs associated with splicing. Detection of such target RNAs can be used as internal standards to normalize amounts of input RNA. Controls can also include HARP mimics which lack cleavable regions, but are amplification competent.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there is more than one component in the kit (labeling reagent and label may be packaged together), the kit also will generally contain a second, third or other additional container into which the additional components may be separately placed. However, various combinations of components may be comprised in a vial. The kits of the present invention also will typically include a means for containing the nucleic acids, and any other reagent containers in close confinement for commercial sale. Such containers may include injection or blow-molded plastic containers into which the desired vials are retained.

When the components of the kit are provided in one and/or more liquid solutions, the liquid solution is an aqueous solution, with an aqueous solution being particularly preferred.

However, the components of the kit may be provided as dried powder(s). When reagents and/or components are provided as a dry powder, the powder can be reconstituted by the addition of a suitable solvent. It is envisioned that the solvent may also be provided in another container means. It is contemplated that 10, 20, 30, 40, 50, 60, 70, 80, 90, 100, 120, 120, 130, 140, 150, 160, 170, 180, 190, 200, 300, 400, 500, 600, 700, 800, 900, 1000 µg or at least or at most those amounts of dried dye are provided in kits of the invention. The dye may then be resuspended in any suitable solvent, such as DMSO.

The container means will generally include at least one vial, test tube, flask, bottle, syringe and/or other container means, into which the nucleic acid formulations are placed, preferably, suitably allocated. The kits may also comprise a second container means for containing a sterile, pharmaceutically acceptable buffer and/or other diluent.

The kits of the present invention will also typically include a means for containing the vials in close confinement for commercial sale, such as, *e.g.,* injection and/or blow-molded plastic containers into which the desired vials are retained.

Such kits may also include components that facilitate isolation of the labeled miRNA. It may also include components that preserve or maintain the miRNA or that protect against its degradation. Such components may be RNase-free or protect against RNases. Such kits generally will comprise, in suitable means, distinct containers for each individual reagent or solution.

A kit will also include instructions for employing the kit components as well the use of any other reagent not included in the kit. Instructions may include variations that can be implemented.

Kits of the invention may also include one or more of the following: Control RNA; nuclease-free water; RNase-free containers, such as 1.5 ml tubes; RNase-free elution tubes; ethanol; acetic acid; sodium acetate; ammonium acetate; guanidinium; detergent; nucleic acid size marker; RNase-free tube tips; and RNase or DNase inhibitors.

It is contemplated that such reagents are embodiments of kits of the invention. Such kits, however, are not limited to the particular items identified above and may include any reagent used for the manipulation or characterization of miRNA or of other target nucleic acid.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Unless otherwise designated, catalog numbers refer to products available by that number from Ambion, Inc.®, The RNA Company.

### EXAMPLE 1

### Detection of miRNA target using HARP assay in qPCR™ format

Detection of target nucleic acid using the method of the present invention was carried out according to the following protocol.

A HARP probe was designed to detect a brain-specific miRNA (miR-124) and consisted of a 71 base chimeric oligonucleotide having the following sequence:

The HARP probe is chimeric in the sense that is contains DNA bases and RNA bases (RNA bases are shown in lowercase). The features of the HARP probe, from 5' to 3', are a first Amplification Region (position 1 - 19 comprising a Forward primer binding site); a Detection Region (position 20 - 34 comprising a 15 base TAQMAN probe-binding site); a 22 base Target Hybridization Region (position 26 - 47, underlined, comprising 22 bases that are complementary to miRNA); a Probe Inactivation Region positioned within the Target Hybridization Region (position 40 - 42 comprising 3 bases of RNA, shown in lower case) and a second Amplification Region (position 38 - 57 comprising a Reverse primer binding site). The RNA bases comprise a region that can be cleaved by ribonuclease when it is not hybridized to target nucleic acid. Note that the 3' side of the Detection Region overlaps the 5' side of the Target Hybridization Region by 9 bases.

The assay was carried out as follows:
1. Total RNA (200 ng) extracted from mouse brain was mixed with 1 fmole of HARP probe in a volume of 3 ul. As a negative control, 200 ng of mouse liver was mixed with 1 fmole of HARP probe in a volume of 3 µl. The total RNA was extracted using a modified silica filter method designed to recover very small RNA molecules, including miRNAs. The RNA was quantified via UV absorbance. The RNA and HARP probes were in nuclease-free water containing 0.1 mM EDTA pH 8.
2. 3 µl of hybridization buffer was added to the [RNA + probe] and mixed by vortexing. The composition of the hybridization buffer was: 80% Formamide, 300 mM NaOAc, 100 mM NaCitrate, 1 mM EDTA, and 5 % PEG.
3. Target and probe were incubated at 37°C for 20 hours
4. 14 µl of RNase digestion mix was then added and the reactions were incubated for 45 min. at 37°C. The composition of RNase digestion mix was: 10 µl of 280 mM KCl, 0.33 µl of 100 µ/µl RNase 1, 0.5 µl RNase A (.25 µ/µl) /RNase T1 (10 µ/µl), 3.17 µl nuclease-free dH₂O. (Note: the amount of RNase added can be adjusted for detection of other targets according to the mass and sequence of target RNA and mass amount of HARP probe used, and according to the sequence and size of the RNA region within the HARP probe.)
5. 20 µg of proteinase K was added in a volume of 1 µL to inactivate the RNase and the reaction was incubated at 37°C for 10 minutes.
6. The digestion was heated at 70°C for 10 minutes in an ABI 9600 thermalcycler.
7. 5 µl of the above reaction was added to 20 µl of PCR™ Master Mix containing Forward and Reverse PCR™ primers (400 nM final concentration each) and 1 unit of Taq polymerase. The reaction was cycled according to the following program on the ABI 7000 real-time thermalcycler instrument : 95°C/3 minutes // 40 cycles (95°C/ 15 secs// 55°C/10 secs// 72°C/ 35 secs).
TAQMAN probe: 5'- CTCAAGTGGCATTCA - 3' (SEQ ID NO:5)
Forward primer: 5' - CTTTTCCCGTCCGTCATCG - 3' (SEQ ID NO:6)
Reverse primer: 5' - CTA TAG GGA GTT AAG GCA CG - 3' (SEQ ID NO:7)

The amplification plot resulting from this study showed detection of the miR-124 target with Ct values of 20.08 and 21.01 in the technical duplicate reactions. The mouse liver (minus-target) plus-RNase control was undetected, indicating complete cleavage of the unhybridized HARP probe.

### EXAMPLE 2

### Detection of miRNA target using HARP assay in qRT-PCR format

To demonstrate the use of a HARP probe to detect miRNA in a qRT-PCR format, a HARP probe was designed with additional RNA bases, requiring a reverse transcription of 10 RNA bases. The HARP probe served the same purpose: to detect a brain-specific miRNA (miR-124) and consisted of a 71 base chimeric oligonucleotide having the following sequence (RNA bases shown in lower case):

The primary features of the HARP probe, from 5' to 3', are a first Amplification Region (position 1 - 18 comprising a Forward primer binding site); a Detection Region (position 19 - 37 comprising a 19 base TAQMAN probe-binding site); a 22 base Target Hybridization Region (position 26 - 47, underlined, comprising 22 bases that are complementary to miRNA); a Probe Inactivation Region (position 29 - 38 comprising 10 bases of RNA, shown in lower case) and a second Amplification Region (position 38 - 57 comprising a Reverse primer binding site). The RNA bases comprise a region that can be cleaved by ribonuclease when it is not hybridized to target nucleic acid. Note that the 3' side of the Detection Region overlaps the 5' side of the Target Hybridization Region by 12 bases. The assay was carried out as follows:
1. Total RNA (1 µg) extracted from mouse brain was mixed with 1 fmole of HARP probe in a volume of 3 µl. As a negative control, 1 µg of mouse liver was mixed with 1 fmole of HARP probe in a volume of 3 µl. The total RNA was extracted using a modified silica filter method designed to recover very small RNA molecules, including miRNAs. The RNA was quantified via UV absorbance. The RNA and HARP probes were in nuclease-free water containing 0.1 mM EDTA pH 8.
2. 3 µl of hybridization buffer was added to the [RNA + probe] and mixed by vortexing. The composition of the hybridization buffer was: 80% Formamide, 300 mM NaOAc, 100 mM NaCitrate, 1 mM EDTA, and 5 % PEG.
3. Target and probe were incubated at 37°C for 20 hours
4. 14 µl of RNase digestion mix was then added and the reactions were incubated for 30 min. at 37°C. The composition of the RNase digestion mix was: 10 µl of 280 mM KCl, 0.33 µl of 100 µ/µl RNase 1, 0.5 µl RNase A (.25 µ/µl) /RNase T1 (10 µ/µl), 3.17 µl nuclease-free dH₂O. (Note: the amount of RNase added can be adjusted for detection of other targets according to the mass and sequence of target RNA and mass amount of HARP probe used, and according to the sequence and size of the RNA region within the HARP probe.)
5. 20 µg of proteinase K and 10 picomoles of reverse primer were added in a volume of 2 µL to inactivate the RNase and the reaction was incubated at 37°C for 10 minutes.
6. The digestion was heated at 70°C for 10 minutes then cooled to 4°C in an ABI 9600 thermalcycler.
7. 5 µl of the above reaction was added to 20 µl of RT-PCR Master Mix containing Forward and Reverse PCR™ primers (400 nM final concentration each), 10 units of MMLV reverse transcriptase, 12 units of placental RNase inhibitor, and 1 unit of Taq polymerase. The reaction was cycled on the ABI 7000 real-time thermalcycler instrument as follows:
   42°C/15 min // 95°C/ 10 minutes // 40 cycles (95°C/ 15 secs/ 60°C/ 40 secs).
   TAQMAN probe: 5'- GCTCAAGTGGCATTCACCG - 3' (SEQ ID NO:9)
   Forward: 5' - CTTTTCCCGTCCGTCATC - 3' (SEQ ID NO:10)
   Reverse: 5' - CTA TAG GGA GTT AAG GCA CG - 3' (SEQ ID NO:11)

The results of this study showed detection of the miR-124 target with Ct values of (15.07 and 15.11)n technical duplicate reactions. The mouse liver (minus-target) plus-RNase control was undetected, indicating complete cleavage of the unhybridized HARP probe. This study, therefore, demonstrates the use of a HARP probe to detect miRNA in a qRT-PCR format.

### EXAMPLE 3

### Sensitivity and linearity for detection of miRNA in human sample

To demonstrate the sensitivity and linearity of detection of miRNA in a human sample, a HARP assay was carried out as described in Example 1, except that HARP probe was hybridized overnight using 3 different amounts of total RNA (100 ng, 10 ng, and 1 ng) from human brain as target. The miR-124 target was detected with Ct values shown in Table 1 below. Note, a difference of 3.3 between two Ct values reflects a 10-fold difference in amount of target. This study demonstrates linear detection of the miR-124 target RNA between 100 ng and 1 ng; the minus-target-plus RNase control reaction did not show any amplification, showing that amplification of the HARP probe was target-dependent.

**Table 1**

| Amount of human brain RNA input | Ct value for miR-124detection via HARP assay |
|---|---|
| 100 ng | 21.74 |
| 10 ng | 25.24 |
| 1 ng | 28.84 |
| None | not detected |

### EXAMPLE 4

### Detection of mRNA target and discrimination of related sequences using the HARP assay

The HARP assay is also useful for detecting messenger RNA targets in biological samples. The HARP assay was used to detect mRNA for a ubiquitously-expressed gene, human GAPDH (glyceraldehyde phosphate dehydrogenase). The HARP probe contained 15 bases of RNA and was designed to hybridize to positions 258 - 277 of human GAPDH mRNA (Accession # NM_002046 ) This region of human GAPDH mRNA has 2 sequence differences (a T>C transition at position 261 and a C>T transition at position 267) compared to the corresponding region of mouse mRNA for GAPDH. One fmole of HARP probe was hybridized overnight to 1 µg of total RNA from mouse or human brain, then the reactions were treated with RNases A/T1/1 to cleave unhybridized HARP probe. The RNase was inactivated by protease digestion and protected HARP probe amplified and detected by qRT-PCR as described in Example 2. Table 2 below shows detection of mRNA for GAPDH in total RNA extracted from human brain but not from mouse brain. Not wishing to be bound by theory, the inventors believe that these results are due to cleavage of the human sequence HARP probe hybridized to mouse RNA with the 2 mismatches. No amplification of the HARP probe was detected after RNase digestion in the absence of target RNA.

**Table 2**

| Source of input RNA | Ct value for detection of GAPDH mRNA |
|---|---|
| human RNA | 23.96, 24.05 (technical duplicates) |
| mouse RNA | Not detected |
| None | Not detected |

### EXAMPLE 5

### Sensitivity and specificity are maintained in HARP assay using multiplex (2 HARP probes) hybridization and RNase digestion format

One microgram of human brain RNA was hybridized overnight with 1 fmole of HARP probe for GAPDH and 1 fmole of HARP for miR-124, or with 1 fmole of miR-124 HARP alone. Control samples contained HARP probes with no target RNA. After hybridization the samples were treated with RNases A/T1/1 and used as input for qRT-PCR as described for Example 1. The protected HARP probe for miR-124 was amplified with equal efficiency whether hybridized alone or with the second HARP, and there was no amplification in the minus-target control reactions. In the corresponding experiment using a HARP probe for human GAPDH, one microgram of human brain RNA was used in the HARP assay as described in Example 7, except that the hybridization reaction contained only a single HARP for detection of human GAPDH mRNA. As set forth in Table 3, the Ct value is identical to that observed when the hybridization reaction contained a second HARP probe for miR-124. In a related experiment to assess tissue specificity for detection using the HARP assay in a multiplex format, one microgram of human liver RNA was hybridized to 1 fmole of HARP probe for human GAPDH and 1 fmole of HARP probe for mir-124 and the HARP assay was carried out as described in Example 1. The qPCR showed no detection of miR-124 in liver, which is the expected result since miR-124 is known to be specifically expressed in brain. This result demonstrates that 2 different HARPs can be combined in a hybridization/RNase digestion reaction without causing non-specific protection and amplification.

**Table 3**

| HARP probe | target RNA | Ct values (technical duplicates) |
|---|---|---|
| miR-124 and human GAPDH | 1 µg human brain | 20, 19.61 (for miR-124) |
| miR-124 | 1 µg human brain | 19.69, 19.28 (for miR-124) |
| miR-124 and human GAPDH | None | not detected (miR-124) |
| human GAPDH | 1 µg human brain | 23.75, 23.60 (for GAPDH) |
| human GAPDH and miR-124 | 1 µg human brain | 23.01, 23.00 (for GAPDH) |
| human GAPDH and miR-124 | None | not detected (GAPDH) |
| human GAPDH and miR-124 | 1 µg human liver | not detected (miR-124) |
| human GAPDH and miR-124 | 1 µg human liver | 24.38, 24.36 (for GAPDH) |

### EXAMPLE 6

### Technical sensitivity and linearity for amplifying uncleaved HARP probe.

A 10-fold dilution series was prepared for a HARP probe for miR-124 and varying amounts ranging from 50 attomoles (-30,000,000 copies) to 0.5 zeptomoles (∼ 300 copies) was used as input for a series of mock HARP assays (no target, no RNase) and corresponding PCR™ reactions. Amplification of the HARP probe was carried out as described in Example 1. The Probe Inactivation Region consisted of 3 bases of RNA positioned near the 3' end of the Target Hybridization Region and the Reverse primer spanned this region, so that an RT step was not needed. The amplification plot showed quantitative linear detection of the amplified products over a 6-log range.

### EXAMPLE 7

### Use of HARP assay to discriminate between related DNA targets

A particular advantage of the HARP assay is that it allows discrimination between target mRNAs with related sequences. Since the single-strand-specific RNases used in the HARP assay are able to cleave not only unhybridized RNA bases, but also RNA bases duplexed with non-complementary bases *(i.e.* "basepair mismatches"), these RNases are able to cleave, and thus discriminate against, HARP probes that are hybridized to sequences related to but different from the perfectly complementary target sequences.

This capability was illustrated by a study, which shows the ability of the HARP assay to discriminate targets that differ by a *single* base. A HARP probe for miR-124 was used, that contained 3 bases of RNA near the 3' end of the Hybridization Region. Two synthetic target DNA oligonucleotides of 22 bases were designed, one that was perfectly complementary to the Target Hybridization Region, and one that had a single G>C sequence difference in the Probe Inactivation Region (sequences are shown below).
No-mismatch oligo:
5' TTA AGG CAC GCG GTG AAT GCC A 3' (SEQ. ID NO:12)
Mismatch oligo:
5' TTA AGG CAC GCG GTC AAT GCC A 3' (SEQ. ID NO:13)

The 2 oligos were used as targets in the HARP assay. 100 amoles of the synthetic targets were hybridized to 1 fmole of mir 124 HARP probe overnight in a background of 100 ng of yeast RNA. The samples were then digested for one hour at 37°C with 14 µl digestion mix consisting of 10 µl 280 mM KCl, 1 µl RNase 1 (100 µ/µl) and 0.5 µl RNase A (.25 µ/µl) /RNase T1 (10 µ/µl) and 3.5 µl nuclease-free dH₂O. The RNases were inactivated and 5 µl of each sample served as input for a 40 cycle one-step qPCR. The Ct values for three different samples, each run in duplicate, were as follows:
a) no RNase control (250 amoles undigested HARP probe), Ct: 15.67, 15.68
b) 250 amoles HARP probe protected with 25 amoles no-mismatch target oligo, Ct: 24.03, 23.58
c) 250 amoles HARP probe protected with 25 amoles mismatched target oligo, Ct: undetermined

### EXAMPLE 8

### Detection of target sequences via HARP assay using in vitro transcription as the amplification method

Amplification methods other than PCR™ can be used to detect target sequences using the HARP assay. One such method is by *in vitro* transcription of protected HARP probes using phage RNA polymerases. This method requires that the HARP probe be designed to include phage promoter sequences, for example the promoter for T7 phage RNA polymerase or its complementary sequence.

For detection of protected HARP probes, without prior amplification by PCR™, the Amplification Region consisted of the reverse complement of the T7 promoter incorporated at the 3' end of the HARP probe. The HARP probe was designed to detect miR-124. After hybridization of the HARP probe to various amounts of synthetic miR-124 target RNA and RNase cleavage, the reaction was treated with proteinase K to inactivate the RNase; this step is important to avoid RNase digestion of the *in vitro* transcription product. After the protease step, a molar excess of an oligonucleotide having the T7 promoter top-strand consensus sequence (5'-TAATACGACTCACTATAGGG 3' (SEQ. ID NO:14) was hybridized to the reaction products to form a functional double-stranded T7 promoter, and the resulting reaction products were used as template for *in vitro* transcription with T7 RNA polymerase. The RNA transcript was labeled by incorporation of UTP radioisotopically labeled in the alpha-position with ³²P. The labeled RNA transcript was detected by autoradiography after size separation of the reaction products by electrophoresis on a denaturing polyacrylamide gel.

The autoradiograph showed detection of the protected HARP probe by transcription with T7 RNA polymerase. *The in vitro* transcription product was only seen in the presence of target RNA and T7 polymerase. The *in vitro* transcription product was not seen in the absence of target RNA or of T7 polymerase or of the T7 top strand oligonucleotide.

### EXAMPLE 9

### Amplification by Non-PCR Methods

As discussed above, amplification methods other than PCR™ can be used to detect target sequences using the HARP assay.

One alternative amplification method is the Strand Displacement Amplification (SDA). In this method the HARP probe can be synthesized with HincII restriction sites on both ends of the HARP probe, flanking the Target Hybridization Region. The HincII site on the 3' end of the HARP probe can have, for example, the sequence C-A As-C-T-G where "As" is defined as a phosphorothiorate linkage which is refractory to cleavage by Hinc II. After hybridization of the HARP probe to target RNA, hybridization and RNase cleavage, the reaction can be treated with proteinase K to inactivate the RNase. After the protease step, SDA primers can be added. The sequence of the primers and the reaction conditions can be, for example, similar to those described in (Walker *et al,* 1992). The reaction conditions would typically differ in that 0.1-0.5 mM MgCl₂ will be added to assist the Exo-klenow polymerase in reading through the RNA region of the HARP probe. This will cause an exponential amplification of the HARP probe.

Alternatively, one may bring about amplification in the context of the invention by TMA or NASBA. Those of skill will, in view of the teachings of this specification, be able to design appropriate protocols to accomplish these embodiments of the invention and any other embodiments of the invention that rely upon any other amplification protocols that are known at the time of filing, or later discovered.

### EXAMPLE 10

### "Direct Protect" Embodiments of the Invention

An alternative rapid HARP assay strategy can utilize a lysis buffer that would also support hybridization. A similar strategy is used in the Ambion "Direct Protect Kit," which allows detection of target RNA in crude biological samples without prior purification of total RNA. In such a protocol, after cell lysis and hybridization to HARP probe, the lysis buffer would be diluted and the sample digested with RNase. HARP probes that were hybridized to target would be protected and free probe would be cleaved. Uncleaved HARP probe would be collected for amplification. In the "Direct Protect" procedure the products of the RNase digestion are concentrated by ethanol precipitation. While this strategy will work well in the HARP context, it is laborious. An alternative would be to tag the HARP probes with a tag that would allow capture of the HARP from the RNase digestion reaction. This might be done with a biotin or other group that will allow capture. The HARP probe could then be captured on streptavidin beads or other appropriate solid supports, washed and moved to an *in vitro* transcription reaction. This would be expected to give a 1000 fold or greater amplification. (Since the 3' end of the transcripts is a defined sequence, the amplification products could be easily amplified in a second round to yield a million fold amplification if desired.) Alternatively, with PCR™ HARPs, the HARP probe recovered on beads could be amplified by PCR™. In a multiplex mode, the amplification products can then be hybridized to an array for quantification.

### EXAMPLE 11

### Multiplex HARP detection by different sized transcription products

HARP probes can be used in a multiplex format. In a relatively simple embodiment, HARP probes can be synthesized that differ in size, so that the *in vitro* transcription products differ in size. The reactions would contain a detectable label such as ³²P nucleotide or dye labeled nucleotide. This would allow different HARP amplification products to be distinguished from each other by size after fractionation, for example on polyacrylamide or agarose gels or by HPLC, capillary electrophoresis, or other nucleic acid sizing technique. This approach would easily allow 5-10 different HARP probes to be distinguished. While this approach could be used with PCR™ HARP probes, this approach would be less than ideal since the longer probes would be expected to amplify less efficiently than the shorter probes. This would not be an issue with linear amplification approaches such as *in vitro* transcription.

### EXAMPLE 12

### Multiplex HARP assay using HARP probes on solid supports

HARP assays can be carried out in a highly multiplexed format by hybridizing multiple HARP probes to a biological sample potentially containing multiple different complementary RNA and/or DNA targets. After hybridization, the mixture of probes would be treated (for example with single-strand-specific RNase(s)) to eliminate those that were not hybridized to target, and the protected probes could be amplified and then detected and distinguished by hybridization to complementary features on a microarray.

For this form of a multiplex application, it would be useful to introduce a detectable label during the amplification step, for example by using Forward and/or Reverse PCR™ primers modified with biotin residues or with fluorescent dyes on their 5' ends. The amplification products would then be hybridized to an array that contained capture sequences complementary to each of the amplification products. This would allow quantification of many different sequences from one reaction. Alternatively the amplification products could be captured on a non-conventional array. An example of this is the Luminex bead arrays. It might be useful to include in the design of the HARP probe. Detection Regions comprising "Zip code" sequences that would allow a universal array to be used to capture the amplification products from a HARP probe. If there were a small number of different probes, they could be quantified by having the probes generate amplification products that differed in size. These could be distinguished and quantified by gel electrophoresis or HPLC.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

U.S. Patent 4,682,195
U.S. Patent 4,683,202
U.S. Patent 4,800,159
U.S. Patent 4,876,187
U.S. Patent 4,946,773
U.S. Patent 5,011,769
U.S. Patent 5,011,769
U.S. Patent 5,480,980
U.S. Patent 5,637,683
U.S. Patent 5,660,988
U.S. Patent 5,660,988
U.S. Patent 5,723,591
U.S. Patent 5,723,591
U.S. Patent 5,728,525
U.S. Patent 5,801,155
U.S. Patent 5,843,650
U.S. Patent 5,846,709
U.S. Patent 5,846,783
U.S. Patent 5,849,483
U.S. Patent 5,849,497
U.S. Patent 5,849,546
U.S. Patent 5,849,547
U.S. Patent 5,851,770
U.S. Patent 5,858,652
U.S. Patent 5,866,337
U.S. Patent 5,866,366
U.S. Patent 5,882,864
U.S. Patent 5,916,776
U.S. Patent 5,916,779
U.S. Patent 5,922,574
U.S. Patent 5,925,525
U.S. Patent 5,928,870
U.S. Patent 5,928,905
U.S. Patent 5,928,906
U.S. Patent 5,932,451
U.S. Patent 5,935,825
U.S. Patent 5,939,291
U.S. Patent 5,942,391
U.S. Patent 6,084,102
U.S. Patent 6,174,670
U.S. Patent 6,232,066
U.S. Patent 6,238,869
U.S. Patent 6,251,666
U.S. Patent 6,458,533
U.S. Patent No. 6,706,480
U.S. Patent Appln. 20030170623
U.S. Patent Appln. 2003/0096232
U.S. Serial No. 10/462,091
U.S. Serial No. 10/899,386
U.S. Serial No. 10/667,126
U. S. Prov Patent Appln. 60/575,743
U. S. Prov Patent Appln. 60/575,743
Armour et al., Nucleic Acids Res., 28(2):605-609, 2000.
Ausubel et al., In: Current Protocols in Molecular Biology, John, Wiley & Sons, Inc, New York, 1994.
Bellus, J Macromol. Sci. Pure Appl. Chem., A31(1): 1355-1376, 1994.
Carrington et al., Science, 301(5631):336-338, 2003.
Cummins et al., In: IRT: Nucleosides and nucleosides, La Jolla CA, 72, 1996.
European Appln. 329 822
Great Britain Appln. 2 202 328
Griffey et al., J. Mass Spectrom, 32(3):305-13, 1997.
Hardenbol et al., Nat. Biotechnol., 21(6):673-678, 2003.
Innis et al., Proc. Natl. Acad. Sci. USA, 85(24):9436-9440, 1988.
Kim et al., Proc. Natl. Acad. Sci. USA, 101:360-365, 2004.
Kornberg and Baker, DNA Replication, 2nd Ed., Freeman, San Francisco, 1992.
Kwoh et al., Proc. Natl. Acad. Sci. USA, 86:1173, 1989.
Pasquinelli and Ruvkun, Ann. Rev. Cell Dev. Biol., 18:495-513, 2002.
PCT Appln. PCT/US87/00880
PCT Appln. PCT/US89/01025
PCT Appln. WO 03022421
PCT Appln. WO 03023058
PCT Appln. WO 03029485
PCT Appln. WO 03066906
PCT Appln. WO 03067217
PCT Appln. WO 03076928
PCT Appln. WO 03093810
PCT Appln. WO 03100448A1
PCT Appln. WO 88/10315
PCT Appln. WO 89/06700
PCT Appln. WO 90/07641
PCT Appln. WO 95/21944
PCT Appln. WO 9743450
Sambrook et al., In: Molecular cloning, Cold Spring Harbor Laboratory Press, Cold Spring Harbor, NY, 1989.
Sambrook et al., In: Molecular cloning, Cold Spring Harbor Laboratory Press, Cold Spring Harbor, NY, 2001.
Sambrook et al., In: Molecular cloning, Cold Spring Harbor Laboratory Press, Cold Spring Harbor, NY, 1989.
Scheit, In: Synthesis and Biological Function, Wiley-Interscience, New York, 171-172, 1980.
Schouten et al., Nucleic Acids Res., 30(12):e57, 2002.
Smooker and Cotton, Mutation Research, 288:65-77, 1993
Walker et al, Proc. Natl. Acad. Sci. USA, 89:392-296, 1992.
Walker et al., Nucleic Acids Res., 20(7):1691-1696, 1992.

## Claims

1. A method for amplifying a target nucleic acid sequence comprising:
contacting, under hybridization conditions, a nucleic acid comprising the target nucleic acid sequence with a chimeric probe comprising RNA and DNA ("HARP probe") comprising a region, which hybridizes to the target nucleic acid ("Target Hybridization Region") and at least one region comprising one or more segments that can be used to amplify all or part of the HARP probe ("Amplification Region");
exposing the HARP probe to one or more RNase(s) that render the HARP probe non-amplifiable when there is no hybridization between the HARP probe and the target nucleic acid sequence; and
amplifying the sequence complementary to at least the Target Hybridization Region of the HARP probe using one or more Amplification Regions;
wherein all or part of the HARP probe is amplified.

2. The method of claim 1, **characterized in that** the HARP probe comprises an Amplification Region that comprises a promoter sequence.

3. The method of claim 2, **characterized in that** the promoter sequence is a T3, T7, or SP6 promoter sequence.

4. The method of any of claims 1 to 3, **characterized in that** the HARP probe comprises at least two Amplification Regions comprising sequences for hybridization of amplification primers to permit amplification using PCR™.

5. The method of any of claims 1 to 4, **characterized in that** the Target Hybridization Region is between 1 and 100 residues in length, preferably between 10 and 50 residues in length, more preferred between 15 and 30 residues in length.

6. The method of any of claims 1 to 5, **characterized in that** the Target Hybridization Region is chimeric.

7. The method of either of claims 5 or 6, **characterized in that** the chimeric HARP probe comprises at least one ribonucleotide and contiguous deoxyribonucleotides.

8. The method of any of claims 5 to 7, **characterized in that** the chimeric HARP probe comprises at least a first RNA portion and at least a first DNA portion.

9. The method of claim 8, **characterized in that** the Target Hybridization Region comprises all or part of the first RNA portion.

10. The method of either of claims 8 or 9, **characterized in that** the chimeric HARP probe comprises one RNA portion and one DNA portion.

11. The method of claim 10, **characterized in that** the Target Hybridization Region comprises RNA and DNA.

12. The method of claim 11, **characterized in that** the chimeric HARP probe has the DNA portion followed by the RNA portion.

13. The method of any of claims 8 to 12, **characterized in that** the first DNA portion comprises at least one Amplification Region.

14. The method of any of claims 8 to 13, **characterized in that** the first DNA portion further comprises part of the Target Hybridization Region.

15. The method of any of claims 8 to 13, **characterized in that** the first Amplification Region includes a promoter sequence.

16. The method of any of claims 8 to 13, **characterized in that** the chimeric HARP probe further comprises a second DNA portion, preferably a DNA portion, then an RNA portion, then a DNA portion.

17. The method of any of claims 8 to 13, **characterized in that** the HARP probe comprises a second DNA region that includes a second Amplification Region, preferably the Amplification Regions are adapted for use for exponential amplification.

18. The method of claim 17, **characterized in that** the first and second DNA portions flank a RNA region.

19. The method of any of claims 5 to 18, **characterized in that** the chimeric HARP probe comprises a DNA portion and a DNA portion comprising nucleotide analogs, wherein preferably the DNA portion comprising nucleotide analogs is resistant to modification with the RNase(s), or wherein preferably the DNA portion comprising nucleotide analogs is not susceptible to modification with the RNase(s) when unhybridized.

20. The method of any of claims 5 to 19, **characterized in that** the RNA portion fully complements all or part of the target nucleic acid sequence.

21. The method of any of claims 1 to 20, **characterized in that** the HARP probe is amplified using a promoter sequence for in vitro transcription.

22. The method of any of claims 1 to 21, **characterized in that** the HARP probe is amplified using PCR™, preferably in a buffer comprising KCl.

23. The method of any of claims 1 to 22, **characterized in that** the RNase(s) modifies the HARP probe by cleaving it, preferably by enzymatically cleaving the HARP probe, even more preferred wherein the RNase(s) is a nuclease selected from the group consisting of S1 nuclease, RNase A, RNase T1, and RNase 1.

24. The method of any of claims 1 to 23, further comprising inactivating the RNase prior to amplifying the HARP probe.

25. The method of any of claims 1 to 24, **characterized in that** a proteinase is used to inactivate the RNase.

26. The method of any of claims 1 to 25, **characterized in that** the HARP probe is between 30 and 500 residues in length, preferably between 50 and 100 residues in length.

27. The method of any of claims 1 to 26, further comprising detecting the amplified HARP probes.

28. The method of any of claims 1 to 27, further comprising quantitating the amplified HARP probes.

29. The method of any of claims 1 to 28, **characterized in that** the target nucleic acid sequence is a ribonucleotide sequence, preferably selected from mRNA, rRNA, tRNA, miRNA, or siRNA.

30. A method for amplifying one or more target nucleic acid sequences comprising:
mixing, under hybridization conditions, at least a first nucleic acid molecule comprising the first target nucleic acid sequence with one or more chimeric probes comprising RNA and DNA ("HARP probes") to form a hybridization mixture, wherein the HARP probe comprises a region which hybridizes to the target nucleic acid ("Target Hybridization Region") and at least one region comprising one or more segments that can be used to amplify all or part of the HARP probe ("Amplification Region");
exposing the HARP probe(s) to one or more RNase(s) that modifies modifiable residues in the Target Hybridization Region of the HARP probe(s) if not hybridized to the target nucleic acid; and
amplifying the HARP probe(s).

31. The method of claim 30, **characterized in that** the hybridization mixture comprises a second HARP probe, **characterized in that** the second HARP probe comprises a Target Hybridization Region and at least one Amplification Region.

32. The method of either of claims 30 or 31, **characterized in that** the hybridization mixture comprises a second HARP probe for a second target nucleic acid sequence in the first nucleic acid molecule, wherein the second HARP probe comprises a Target Hybridization Region and at least one Amplification Region.

33. The method of any of claims 30 to 32, further comprising nucleic acid molecules comprising one or more other target nucleic acid sequences and a HARP probe for each of the other nucleic acid sequences.

34. The method of any of claims 30 to 33, **characterized in that** the HARP probes for the nucleic acids have one or two Amplification Regions that is/are distinct from a Target Hybridization Region.

35. The method of any of claims 30 to 34, **characterized in that** the HARP probes for the nucleic acids have the same Amplification Regions.

36. A method for amplifying a target nucleic acid sequence comprising:
contacting, under hybridization conditions, the target nucleic acid sequence with a chimeric probe comprising RNA and DNA ("HARP probe"), wherein the chimeric HARP probe comprises: i) a region which hybridizes to the target nucleic acid ("Target Hybridization Region") that is comprised of DNA nucleotides and of a region that contains nucleotides that can be modified to prevent amplification of the HARP probe ("Probe Inactivation Region") composed of RNA nucleotides and ii) at least one region comprising one or more segments that can be used to amplify all or part of the HARP probe ("Amplification Region") which is composed of DNA;
exposing the chimeric HARP probe to one or more RNases that cleaves RNA comprising the Probe Inactivation Region in the Target Hybridization Region of the HARP probe if not hybridized to the target nucleic acid sequence; and
amplifying at least the sequence complementary to the hybridization sequence of the HARP probe to create an amplified product.

37. The method of claim 36, further comprising detecting and/or quantitating the amplified product.

38. The method of either of claims 36 or 37, **characterized in that** the chimeric HARP probe has at least two amplification domains.

39. The method of any of claims 36 to 38, **characterized in that** the chimeric HARP probe has a DNA portion, followed by an RNA portion, followed by a second DNA portion.

40. The method of any of claims 36 to 39, **characterized in that** the chimeric HARP probe has at least one Amplification Region composed of RNA.

41. The method of any of claims 36 to 40, **characterized in that** the chimeric HARP probe has at least two Amplification Regions, which can be used to hybridize with a pair of PCR™ primers, preferably wherein the chimeric HARP probe further comprises an additional DNA region comprising a Detection Region that comprises a detection probe binding site, preferably being a universal, target-specific or detection probe binding site.

42. The method of claim 41, **characterized in that** the HARP probe is amplified in a method comprising synthesis of its complementary sequence using a First-strand primer that traverses the Probe Inactivation Region.

43. The method of either of claims 41 or 42, **characterized in that** the Probe Inactivation Region comprises RNA.

44. The method of any of claims 41 to 43, **characterized in that** the 3' end of the First-strand primer that traverses the Probe Inactivation Region is complementary to the base at the 5' end of the Probe Inactivation Region or complementary to the first base adjacent to the base on the 5' end of the Probe Inactivation Region or complementary to the second base adjacent to the base on the 5' end of the Probe Inactivation Region.

45. The method of either of claims 41 or 42, **characterized in that** the First-strand primer that traverses the Probe Inactivation Region contains additional nucleotides at the 5' side which are not complementary to any bases in the HARP probe, but which are identical to bases comprising the 3' side of a Universal Reverse primer used to amplify the HARP probe in subsequent PCR™ cycles or other amplification methods.

46. The method of claim 45, **characterized in that** the First-strand primer that traverses the Probe Inactivation Region contains additional nucleotides at the 5' side which are identical to bases comprising the entire Universal Reverse primer used to amplify the HARP probe in subsequent PCR™ cycles or other amplification methods.

## Patentansprüche

1. Verfahren zum Amplifizieren einer Target-Nukleinsäure-Sequenz, das die Schritte umfasst, daß man
- unter Hybridisierungs-Bedingungen eine Nukleinsäure, die die Target-Nukleinsäure-Sequenz umfasst, mit einer chimären Sonde in Kontakt bringt, die RNS und DNS ("HARP-Sonde") umfasst, die einen Bereich, der mit der Target-Nukleinsäure ("Target-Hybridisierungs-Bereich") hybridisiert, und wenigstens einen Bereich umfasst, der einen oder mehrere Abschnitt(e) umfaßt, der/die verwendet werden kann/können, um die gesamte oder einen Teil der HARP-Sonde zu amplifizieren ("Amplifikations-Bereich");
- die HARP-Sonde einer oder mehreren RNase(n) aussetzt, welche die HARP-Sonde nicht-amplifizierbar macht/machen, wenn es keine Hybridisierung zwischen der HARP-Sonde und der Target-Nukleinsäure-Sequenz gibt; und
- die Sequenz, die komplementär zu wenigstens dem Target-Hybridisierungs-Bereich der HARP-Sonde ist, unter Verwendung einer oder mehrerer Amplifikations-Bereiche amplifiziert;
worin die gesamte oder ein Teil der HARP-Sonde amplifiziert wird.

2. Verfahren nach Anspruch 1, **dadurch gekennzeichnet, daß** die HARP-Sonde einen Amplifikations-Bereich umfasst, der eine Promotor-Sequenz umfasst.

3. Verfahren nach Anspruch 2, **dadurch gekennzeichnet, daß** die Promotor-Sequenz eine T3-, T7 oder SP6-Promotor-Sequenz ist.

4. Verfahren nach einem der Ansprüche 1 bis 3, **dadurch gekennzeichnet, daß** die HARP-Sonde wenigstens zwei Amplifikations-Bereiche umfasst, die Sequenzen zur Hybridisierung von Amplifikations-Primern umfassen, um eine Amplifikation unter Verwendung von PCR™ zu erlauben.

5. Verfahren nach einem der Ansprüche 1 bis 4, **dadurch gekennzeichnet, daß** der Target-Hybridisierungs-Bereich zwischen 1 und 100 Reste in der Länge aufweist, vorzugsweise zwischen 10 und 50 Reste in der Länge, noch mehr bevorzugt 15 bis 30 Reste in der Länge.

6. Verfahren nach einem der Ansprüche 1 bis 5, **dadurch gekennzeichnet, daß** der Target-Hybridisierungs-Bereich chimär ist.

7. Verfahren nach einem der beiden Ansprüche 5 oder 6, **dadurch gekennzeichnet, daß** die chimäre HARP-Sonde wenigstens ein Ribonukleotid und benachbarte Desoxyribonukleotide umfasst.

8. Verfahren nach einem der Ansprüche 5 bis 7, **dadurch gekennzeichnet, daß** die chimäre HARP-Sonde wenigstens einen ersten RNS-Anteil und wenigstens einen ersten DNS-Anteil umfasst.

9. Verfahren nach Anspruch 8, **dadurch gekennzeichnet, daß** der Target-Hybridisierungs-Bereich den gesamten oder einen Teil des ersten RNS-Anteils umfasst.

10. Verfahren nach einem der beiden Ansprüche 8 oder 9, **dadurch gekennzeichnet, daß** die chimäre HARP-Sonde einen RNS-Anteil und einen DNS-Anteil umfasst.

11. Verfahren nach Anspruch 10, **dadurch gekennzeichnet, daß** der Target-Hybridisierungs-Bereich RNS und DNS umfasst.

12. Verfahren nach Anspruch 11, **dadurch gekennzeichnet, daß** die chimäre HARP-Sonde den DNS-Anteil, gefolgt von dem RNS-Anteil, aufweist.

13. Verfahren nach einem der Ansprüche 8 bis 12, **dadurch gekennzeichnet, daß** der erste DNS-Anteil wenigstens einen Amplifikations-Bereich umfasst.

14. Verfahren nach einem der Ansprüche 8 bis 13, **dadurch gekennzeichnet, daß** der erste DNS-Anteil darüber hinaus einen Teil des Target-Hybridisierungs-Bereichs umfasst.

15. Verfahren nach einem der Ansprüche 8 bis 13, **dadurch gekennzeichnet, daß** der erste Amplifikations-Bereich eine Promotor-Sequenz einschließt.

16. Verfahren nach einem der Ansprüche 8 bis 13, **dadurch gekennzeichnet, daß** die chimäre HARP-Sonde darüber hinaus einen zweiten DNS-Anteil umfasst, vorzugsweise einen DNS-Anteil, dann einen RNS-Anteil, dann einen DNS-Anteil.

17. Verfahren nach einem der Ansprüche 8 bis 13, **dadurch gekennzeichnet, daß** die HARP-Sonde einen zweiten DNS-Bereich umfaßt, der einen zweiten Amplifkations-Bereich einschließt, vorzugweise die Amplifikations-Bereiche für die Verwendung zur exponentiellen Amplifikation angepasst sind.

18. Verfahren nach Anspruch 17, **dadurch gekennzeichnet, daß** die ersten und zweiten DNS-Anteile einen RNS-Bereich flankieren.

19. Verfahren nach einem der Ansprüche 5 bis 18, **dadurch gekennzeichnet, daß** die chimäre HARP-Sonde einen DNS-Anteil und einen DNS-Anteil umfasst, der Nukleotid-Analoge umfasst, worin vorzugsweise der DNS-Anteil, der Nukleotid-Analoge umfasst, resistent ist gegenüber einer Modifikation mit der/den RNase(n), oder worin vorzugsweise der DNS-Anteil, der Nukleotid-Analoge umfasst, nicht empfänglich ist für eine Modifikation mit der/den RNase(n), wenn er unhybridisiert ist.

20. Verfahren nach einem der Ansprüche 5 bis 19, **dadurch gekennzeichnet, daß** der RNS-Bereich die gesamte oder einen Teil der Target-Nuleinsäure-Sequenz völlig komplettiert.

21. Verfahren nach einem der Ansprüche 1 bis 20, **dadurch gekennzeichnet, daß** die HARP-Sonde amplifiziert wird unter Verwendung einer Promotor-Sequenz für *in* vitro-Transkriptionen.

22. Verfahren nach einem der Ansprüche 1 bis 21, **dadurch gekennzeichnet, daß** die HARP-Sonde amplifiziert wird unter Verwendung von PCR^{™}, vorzugsweise in einem Puffer, der KCl umfasst.

23. Verfahren nach einem der Ansprüche 1 bis 22, **dadurch gekennzeichnet, daß** die RNase(n) die HARP-Sonde durch Spalten modifizieren, vorzugsweise durch enzymatisches Spalten der HARP-Sonde, noch mehr bevorzugt, worin die RNase(n) eine Nuklease ist/sind, die gewählt ist aus der Gruppe, die besteht aus S1-Nuklease, RNase A, RNase T1 und RNase 1.

24. Verfahren nach einem der Ansprüche 1 bis 23, welches darüber hinaus die Inaktivierung der RNase vor dem Amplifizieren der HARP-Sonde umfasst.

25. Verfahren nach einem der Ansprüche 1 bis 24, **dadurch gekennzeichnet, daß** eine Proteinase verwendet wird, die RNase zu inaktivieren.

26. Verfahren nach einem der Ansprüche 1 bis 25, **dadurch gekennzeichnet, daß** die HARP-Sonde zwischen 30 bis 500 Reste in der Länge aufweist, vorzugsweise zwischen 50 und 100 Reste in der Länge.

27. Verfahren nach einem der Ansprüche 1 bis 26, welches darüber hinaus das Detektieren der amplifizierten HARP-Sonden umfasst.

28. Verfahren nach einem der Ansprüche 1 bis 27, welches darüber hinaus das Quantifizieren der amplifizierten HARP-Sonden umfasst.

29. Verfahren nach einem der Ansprüche 1 bis 28, **dadurch gekennzeichnet, daß** die Target-Nukleinsäure-Sequenz eine Ribonukleotid-Sequenz ist, vorzugsweise gewählt aus mRNS, rRNS, tRNS, miRNS oder siRNS.

30. Verfahren zum Amplifizieren einer oder mehrerer Target-Nukleinsäure-Sequenzen, welches die Schritte umfasst, daß man:
- unter Hybridisierungs-Bedingungen wenigstens ein erstes Nukleinsäure-Molekül, das die erste Target-Nukleinsäure-Sequenz umfasst, mit einer oder mehreren chimären Sonde(n), die RNS und DNS ("HARP-Sonde") umfasst/umfassen, mischt unter Bildung einer Hybridisierungs-Mischung,
worin die HARP-Sonde einen Bereich, der mit der Target-Nukleinsäure ("Target-Hybridisierungs-Bereich") hybridisiert, und wenigstens einen Bereich umfasst, der einen oder mehrere Abschnitt(e) umfaßt, der/die verwendet werden kann/können, um die gesamte oder einen Teil der HARP-Sonde zu amplifizieren ("Amplifikations-Bereich");
- die HARP-Sonde(n) einer oder mehrerer RNase(n) aussetzt, welche modifizierbare Reste in dem Target-Hybridisierungs-Bereich der HARP-Sonde(n) modifiziert/modifizieren, wenn sie nicht mit der Target-Nukleinsäure hybridisiert sind; und
- die HARP-Sonde(n) amplifiziert.

31. Verfahren nach Anspruch 30, **dadurch gekennzeichnet, daß** die Hybridisierungs-Mischung eine zweite HARP-Sonde umfasst, **dadurch gekennzeichnet, daß** die zweite HARP-Sonde einen Target-Hybridisierungs-Bereich und wenigstens einen Amplifikations-Bereich umfasst.

32. Verfahren nach einem der beiden Ansprüche 30 oder 31, **dadurch gekennzeichnet, daß** die Hybridisierungs-Mischung eine zweite HARP-Sonde für eine zweite Target-Nukleinsäure-Sequenz in dem ersten Nukleinsäure-Molekül umfasst, worin die zweite HARP-Sonde einen Target-Hybridisierungs-Bereich und wenigstens einen Amplifikations-Bereich umfasst.

33. Verfahren nach einem der Ansprüche 30 bis 32, welches darüber hinaus Nukleinsäure-Moleküle umfasst, die eine oder mehrere andere Target-Nukleinsäure-Sequenz(en) und eine HARP-Sonde für jede der anderen Nukleinsäure-Sequenz(en) umfasst.

34. Verfahren nach einem der Ansprüche 30 bis 33, **dadurch gekennzeichnet, daß** die HARP-Sonden für die Nukleinsäuren einen oder zwei Amplifikations-Bereich(e) aufweisen, der/die verschieden ist/sind von einem Target-Hybridisierungs-Bereich.

35. Verfahren nach einem der Ansprüche 30 bis 34, **dadurch gekennzeichnet, daß** die HARP-Sonden für die Nukleinsäuren dieselben Amplifikations-Bereiche aufweisen.

36. Verfahren zum Amplifizieren einer Target-Nukleinsäure-Sequenz, welches die Schritte umfasst, daß man
- unter Hybridisierungs-Bedingungen die Target-Nukleinsäure-Sequenzen mit einer chimären Sonde in Kontakt bringt, die RNS und DNS ("HARP-Sonde") umfasst, worin die chimäre HARP-Sonde umfaßt:
(i) einen Bereich, der mit der Target-Nukleinsäure ("Target-Hybridisierungs-Bereich") hybridisiert, der aus DNS-Nukleotiden und aus einem Bereich, der Nukleotide enthält, besteht, die modifiziert werden können, um die Amplifikation der HARP-Sonde zu verhindern ("Sonden-Inaktivierungs-Bereich"), die aus RNS-Nukleotiden besteht; und
(ii) wenigstens einen Bereich, der einen oder mehrere Abschnitt(e) umfaßt, der/die verwendet werden kann/können, um die gesamte oder einen Teil der HARP-Sonde zu amplifizieren ("Amplifikations-Bereich"), die aus DNS besteht;
- die chimäre HARP-Sonde einer oder mehreren RNase(n) aussetzt, die die RNS, welche den Sonden-Inaktivierungs-Bereich in dem Target-Hybridisierung-Bereich der HARP-Sonde umfasst, spaltet/spalten, wenn sie nicht mit der Target-Nukleinsäure-Sequenz hybridisiert ist; und
- wenigstens die Sequenz komplementär zu der Hybridisierungs-Sequenz der HARP-Sonde amplifiziert, um ein amplifiziertes Produkt zu erzeugen.

37. Verfahren nach Anspruch 36, welches darüber hinaus das Detektieren und/oder das Quantifizieren des amplifizierten Produkts umfasst.

38. Verfahren nach einem der beiden Ansprüche 36 oder 37, **dadurch gekennzeichnet, daß** die chimäre HARP-Sonde wenigstens zwei Amplifikations-Domänen aufweist.

39. Verfahren nach einem der Ansprüche 36 bis 38, **dadurch gekennzeichnet, daß** die chimäre HARP-Sonde einen DNS-Anteil aufweist, gefolgt von einem RNS-Anteil, gefolgt von einem zweiten DNS-Anteil.

40. Verfahren nach einem der Ansprüche 36 bis 39, **dadurch gekennzeichnet, daß** die chimäre HARP-Sonde wenigstens einen Amplifikations-Bereich aufweist, der aus RNS besteht.

41. Verfahren nach einem der Ansprüche 36 bis 40, **dadurch gekennzeichnet, daß** die chimäre HARP-Sonde wenigstens zwei Amplifikations-Bereiche aufweist, die zum Hybridisieren mit einem Paar von PCR^{™} -Primern verwendet werden können, vorzugsweise worin die chimäre HARP-Sonde darüber hinaus einen zusätzlichen DNS-Bereich umfasst, der einen Detektions-Bereich umfasst, der eine Detektions-Sonden-Bindungsstelle umfasst, die vorzugsweise eine universelle, Target-spezifische oder Detektions-Sonden-Bindungsstelle ist.

42. Verfahren nach Anspruch 41, **dadurch gekennzeichnet, daß** die HARP-Sonde amplifiziert wird in einem Verfahren, welches die Synthese seiner komplementären Sequenz umfasst, unter Verwendung eines First-Strand-Primers, der den Sonden-Inaktivierungs-Bereich verschiebt.

43. Verfahren nach einem der beiden Ansprüche 41 oder 42, **dadurch gekennzeichnet, daß** der Sonden-Inaktivierungs-Bereich RNS umfasst.

44. Verfahren nach einem der Ansprüche 41 bis 43, **dadurch gekennzeichnet, daß** das 3'-Ende des First-Strand-Primers, der den Sonden-Inaktivierungs-Bereich verschiebt, komplementär ist zu der Base an dem 5'-Ende des Sonden-Inaktivierungs-Bereichs, oder komplementär ist zu der ersten Base, die der Base an dem 5'-Ende des Sonden-Inaktivierungs-Bereichs benachbart ist, oder komplementär ist zu der zweiten Base, die der Base an dem 5'-Ende des Sonden-Inaktivierungs-Bereichs benachbart ist.

45. Verfahren nach einem der beiden Ansprüche 41 oder 42, **dadurch gekennzeichnet, daß** der First-Strand-Primer, der den Sonden-Inaktivierungs-Bereich verschiebt, zusätzliche Nukleotide an der 5'-Seite enthält, die nicht komplementär sind zu irgendwelchen Basen in der HARP-Sonde, die jedoch identisch sind zu Basen, die die 3'-Stelle eines Universal-Reverse-Primers umfassen, der verwendet wird, die HARP-Sonde in nachfolgenden PCR^{™}-Zyklen oder anderen Amplifikationsverfahren zu amplifizieren.

46. Verfahren nach Anspruch 45, **dadurch gekennzeichnet, daß** der First-Strand-Primer, der den Sonden-Inaktivierungs-Bereich verschiebt, zusätzliche Nukleotide an der 5'-Seite enthält, die identisch sind zu Basen, die den gesamten Universal-Reverse-Primer umfassen, der verwendet wird, die HARP-Sonde in nachfolgenden PCR^{™}-Zyklen oder anderen Amplifikationsverfahren zu amplifizieren.

## Revendications

1. Procédé d'amplification d'une séquence d'acide nucléique cible comprenant :
- la mise en contact, dans des conditions d'hybridation, d'un acide nucléique comprenant la séquence d'acide nucléique cible avec une sonde chimérique comprenant de l'ARN et de l'ADN (« sonde HARP ») comprenant une région qui s'hybride à l'acide nucléique cible (« Région d'hybridation à la cible ») et au moins une région comprenant un ou plusieurs segments qui peuvent être utilisés pour amplifier la totalité ou une partie de la sonde HARP (« Région d'amplification ») ;
- l'exposition de la sonde HARP à une ou plusieurs RNases qui rendent la sonde HARP non amplifiable lorsqu'il n'y a pas d'hybridation entre la sonde HARP et la séquence d'acide nucléique cible ; et
- l'amplification de la séquence complémentaire à au moins la Région d'hybridation à la cible de la sonde HARP à l'aide d'une ou plusieurs Régions d'amplification ;
la totalité ou une partie de la sonde HARP étant amplifiée.

2. Procédé selon la revendication 1, **caractérisé par le fait que** la sonde HARP comprend une Région d'amplification qui comprend une séquence de promoteur.

3. Procédé selon la revendication 2, **caractérisé par le fait que** la séquence de promoteur est une séquence de promoteur T3, T7 ou SP6.

4. Procédé selon l'une quelconque des revendications 1 à 3, **caractérisé par le fait que** la sonde HARP comprend au moins deux Régions d'amplification comprenant des séquences pour l'hybridation d'amorces d'amplification pour permettre une amplification à l'aide de PCR^{™}.

5. Procédé selon l'une quelconque des revendications 1 à 4, **caractérisé par le fait que** la Région d'hybridation à la cible a une longueur entre 1 et 100 résidus, de préférence entre 10 et 50 résidus, de façon davantage préférée entre 15 et 30 résidus.

6. Procédé selon l'une quelconque des revendications 1 à 5, **caractérisé par le fait que** la Région d'hybridation à la cible est chimérique.

7. Procédé selon l'une ou l'autre des revendications 5 ou 6, **caractérisé par le fait que** la sonde HARP chimérique comprend au moins un ribonucléotide et des désoxyribonucléotides contigus.

8. Procédé selon l'une quelconque des revendications 5 à 7, **caractérisé par le fait que** la sonde HARP chimérique comprend au moins une première partie d'ARN et au moins une première partie d'ADN.

9. Procédé selon la revendication 8, **caractérisé par le fait que** la Région d'hybridation à la cible comprend la totalité ou une partie de la première partie d'ARN.

10. Procédé selon l'une ou l'autre des revendications 8 ou 9, **caractérisé par le fait que** la sonde HARP chimérique comprend une partie d'ARN et une partie d'ADN.

11. Procédé selon la revendication 10,
**caractérisé par le fait que** la Région d'hybridation à la cible comprend de l'ARN et de l'ADN.

12. Procédé selon la revendication 11,
**caractérisé par le fait que** la sonde HARP chimérique a la partie d'ADN suivie par la partie d'ARN.

13. Procédé selon l'une quelconque des revendications 8 à 12, **caractérisé par le fait que** la première partie d'ADN comprend au moins une Région d'amplification.

14. Procédé selon l'une quelconque des revendications 8 à 13, **caractérisé par le fait que** la première partie d'ADN comprend en outre une partie de la Région d'hybridation à la cible.

15. Procédé selon l'une quelconque des revendications 8 à 13, **caractérisé par le fait que** la première Région d'amplification comprend une séquence de promoteur.

16. Procédé selon l'une quelconque des revendications 8 à 13, **caractérisé par le fait que** la sonde HARP chimérique comprend en outre une seconde partie d'ADN, de préférence une partie d'ADN, puis une partie d'ARN, puis une partie d'ADN.

17. Procédé selon l'une quelconque des revendications 8 à 13, **caractérisé par le fait que** la sonde HARP comprend une seconde région d'ADN qui comprend une seconde Région d'amplification, de préférence les Régions d'amplification sont aptes à être utilisées pour une amplification exponentielle.

18. Procédé selon la revendication 17,
**caractérisé par le fait que** des première et seconde parties d'ADN flanquent une région d'ARN.

19. Procédé selon l'une quelconque des revendications 5 à 18, **caractérisé par le fait que** la sonde HARP- chimérique comprend une partie d'ADN et une partie d'ADN comprenant des analogues nucléotidiques, dans lequel de préférence la partie d'ADN comprenant des analogues nucléotidiques est résistante à une modification par la ou les RNases, ou dans lequel, de préférence, la partie d'ADN comprenant des analogues nucléotidiques n'est pas sensible à une modification par la ou les RNases lorsqu'elle est non hybridée.

20. Procédé selon l'une quelconque des revendications 5 à 19, **caractérisé par le fait que** la partie d'ARN complémente entièrement la totalité ou une partie de la séquence d'acide nucléique cible.

21. Procédé selon l'une quelconque des revendications 1 à 20, **caractérisé par le fait que** la sonde HARP- est amplifiée à l'aide d'une séquence de promoteur pour une transcription *in vitro.*

22. Procédé selon l'une quelconque des revendications 1 à 21, **caractérisé par le fait que** la sonde HARP est amplifiée à l'aide d'une PCR^{™}, de préférence dans un tampon comprenant du KC1.

23. Procédé selon l'une quelconque des revendications 1 à 22, **caractérisé par le fait que** la ou les RNases modifient la sonde HARP par clivage de celle-ci, de préférence par clivage enzymatique de la sonde HARP, de façon encore plus préférée dans lequel la ou les Rnases sont une nucléase choisie dans le groupe constitué par la nucléase S1, la RNase A, la RNase T1 et la RNase 1.

24. Procédé selon l'une quelconque des revendications 1 à 23, comprenant en outre l'inactivation de la RNase avant l'amplification de la sonde HARP.

25. Procédé selon l'une quelconque des revendications 1 à 24, **caractérisé par le fait qu'**une protéinase est utilisée pour inactiver la RNase.

26. Procédé selon l'une quelconque des revendications 1 à 25, **caractérisé par le fait que** la sonde HARP a une longueur entre 30 et 500 résidus, de préférence entre 50 et 100 résidus.

27. Procédé selon l'une quelconque des revendications 1 à 26, comprenant en outre la détection des sondes HARP amplifiées.

28. Procédé selon l'une quelconque des revendications 1 à 27, comprenant en outre la quantification des sondes HARP amplifiées.

29. Procédé selon l'une quelconque des revendications 1 à 28, **caractérisé par le fait que** la séquence d'acide nucléique cible est une séquence ribonucléotidique, de préférence choisie parmi l'ARNm, l'ARNr, l'ARNt, l'ARNmi ou l'ARNsi.

30. Procédé d'amplification d'une ou plusieurs séquences d'acide nucléique cible comprenant :
- le mélange, dans des conditions d'hybridation, d'au moins une première molécule d'acide nucléique comprenant la première séquence d'acide nucléique cible avec une ou plusieurs sondes chimériques comprenant de l'ARN et de l'ADN (« sondes HARP ») pour former un mélange d'hybridation, la sonde HARP comprenant une région qui s' hybride à l'acide nucléique cible (« Région d'hybridation à la cible ») et au moins une région comprenant un ou plusieurs segments qui peuvent être utilisés pour amplifier la totalité ou une partie de la sonde HARP (« Région d'amplification ») ;
- l'exposition de la ou des sondes HARP à une ou plusieurs RNases qui modifient des résidus modifiables dans la région d'hybridation à la cible de la ou des sondes HARP si elles ne sont pas hybridées à l'acide nucléique cible ; et
- l'amplification de la ou des sondes HARP.

31. Procédé selon la revendication 30, **caractérisé par le fait que** le mélange d'hybridation comprend une seconde sonde HARP, **caractérisé par le fait que** la seconde sonde HARP comprend une Région d'hybridation à la cible et au moins une Région d'amplification.

32. Procédé selon l'une ou l'autre des revendications 30 ou 31, **caractérisé par le fait que** le mélange d'hybridation comprend une seconde sonde HARP pour une seconde séquence d'acide nucléique cible dans la première molécule d'acide nucléique, la seconde sonde HARP comprenant une Région d'hybridation à la cible et au moins une Région d'amplification.

33. Procédé selon l'une quelconque des revendications 30 à 32, comprenant en outre des molécules d'acide nucléique comprenant une ou plusieurs autres séquences d'acide nucléique cible et une sonde HARP pour chacune des autres séquences d'acide nucléique.

34. Procédé selon l'une quelconque des revendications 30 à 33, **caractérisé par le fait que** les sondes HARP pour les acides nucléiques ont une ou deux Régions d'amplification qui est/sont distinctes d'une Région d'hybridation à la cible.

35. Procédé selon l'une quelconque des revendications 30 à 34, **caractérisé par le fait que** les sondes HARP pour les acides nucléiques ont les mêmes Régions d'amplification.

36. Procédé d'amplification d'une séquence d'acide nucléique cible comprenant :
- la mise en contact, dans des conditions d'hybridation, de la séquence d'acide nucléique cible avec une sonde chimérique comprenant de l'ARN et de l'ADN (« sonde HARP »), la sonde HARP chimérique comprenant : i) une région qui s'hybride à l'acide nucléique cible (« Région d'hybridation à la cible ») qui est composée de nucléotides d'ADN et d'une région qui contient des nucléotides qui peuvent être modifiés pour empêcher une amplification de la sonde HARP (« Région d'inactivation de sonde ») composée de nucléotides d'ARN, et ii) au moins une région comprenant un ou plusieurs segments qui peuvent être utilisés pour amplifier la totalité ou une partie de la sonde HARP (« Région d'amplification ») qui est composée d'ADN ;
- l'exposition de la sonde HARP chimérique à une ou plusieurs RNases qui clivent l'ARN comprenant la Région d'inactivation de sonde dans la Région d'hybridation à la cible de la sonde HARP si elle n'est pas hybridée à la séquence d'acide nucléique cible ; et
- l'amplification d'au moins la séquence complémentaire à la séquence d'hybridation de la sonde HARP pour créer un produit amplifié.

37. Procédé selon la revendication 36, comprenant en outre la détection et/ou la quantification du produit amplifié.

38. Procédé selon l'une ou l'autre des revendications 36 ou 37, **caractérisé par le fait que** la sonde HARP chimérique a au moins deux domaines d'amplification.

39. Procédé selon l'une quelconque des revendications 36 à 38, **caractérisé par le fait que** la sonde HARP chimérique a une partie d'ADN, suivie par une partie d'ARN, suivie par une seconde partie d'ADN.

40. Procédé selon l'une quelconque des revendications 36 à 39, **caractérisé par le fait que** la sonde HARP chimérique a au moins une Région d'amplification composée d'ARN.

41. Procédé selon l'une quelconque des revendications 36 à 40, **caractérisé par le fait que** la sonde HARP chimérique a au moins deux Régions d'amplification, qui peuvent être utilisées pour s'hybrider à une paire d'amorces de PCR^{™}, de préférence dans lequel la sonde HARP chimérique comprend en outre une région d'ADN supplémentaire comprenant une Région de détection qui comprend un site de liaison de sonde de détection, de préférence étant un site de liaison de sonde universel, spécifique de cible ou de détection.

42. Procédé selon la revendication 41, **caractérisé par le fait que** la sonde HARP est amplifiée dans un procédé comprenant la synthèse de sa séquence complémentaire à l'aide d'une Amorce de premier brin qui traverse la Région d'inactivation de sonde.

43. Procédé selon l'une ou l'autre des revendications 41 ou 42, **caractérisé par le fait que** la Région d'inactivation de sonde comprend de l'ARN.

44. Procédé selon l'une quelconque des revendications 41 à 43, **caractérisé par le fait que** l'extrémité 3' de l'Amorce de premier brin qui traverse la Région d'inactivation de sonde est complémentaire à la base à l'extrémité 5' de la Région d'inactivation de sonde ou complémentaire à la première base adjacente à la base sur l'extrémité 5' de la Région d'inactivation de sonde ou complémentaire à la seconde base adjacente à la base sur l'extrémité 5' de la Région d'inactivation de sonde.

45. Procédé selon l'une ou l'autre des revendications 41 ou 42, **caractérisé par le fait que** l'Amorce de premier brin qui traverse la Région d'inactivation de sonde contient des nucléotides supplémentaires à l'extrémité 5' qui ne sont pas complémentaires à n'importe quelles bases dans la sonde HARP, mais qui sont identiques à des bases comprenant l'extrémité 3' d'une Amorce inverse universelle utilisée pour amplifier la sonde HARP dans des cycles de PCR^{™} ultérieurs ou autres procédés d'amplification.

46. Procédé selon la revendication 45, **caractérisé par le fait que** l'Amorce de premier brin qui traverse la Région d'inactivation de sonde contient des nucléotides supplémentaires à l'extrémité 5' qui sont identiques à des bases comprenant l'Amorce inverse universelle entière utilisée pour amplifier la sonde HARP dans des cycles de PCR^{™} ultérieurs ou autres procédés d'amplification.
